# EUROPEAN PATENT APPLICATION

(11) **EP 0 869 176 A1**
(43) Date of publication of application: **07.10.1998**
(21) Application number: 96937511.2
(22) Date of filing: 06.11.1996
(51) Int. Cl.: C12N 15/12, C12P 21/02, C12N 5/10, C07K 14/715, C07K 14/47, G01N 33/53, G01N 33/531

(54) **AUTOANTIGENS**

(30) Priority: 07.11.1995 JP 288957/95
(71) Applicant: KANEKA CORPORATION, Osaka-shi, Osaka 530 (JP)
(72) Inventor: OSAKI, Shoichi, Kyoto-shi,Kyoto 615 (JP); TANAKA, Masao, Sanjo-dori, Nakagyo-ku-Kyoto 604 (JP); KISHIMURA, Masaaki, Kyoto-shi,Kyoto 612 (JP); NAKAO, Kazuwa, Kyoto-shi,Kyoto 610-11 (JP); OSAKADA, Fumio, 2-19-2, Hinode-cho,Himeji-shi,Hyogo 670 (JP)
(74) Representative: Harding, Charles Thomas
(86) International application number: JP9603250
(87) International publication number: WO9717441

(57) **Abstract**

The present invention provides a method for obtaining a novel antigen to which antibodies associated with rheumatoid arthritis (RA) reacts specifically and detecting RA patients by using the antigen, as well as a composition and a kit for the same. cDNA libraries were made from synovial cells, and a screening for the antigen was conducted by using IgG in synovial fluid from RA patients. Thus, a clone A polypeptide, which is a novel polypeptide as a RA antigen, and follistatin related protein (FRP), which is known as a polypeptide but novel as a RA antigen, were isolated. An antibody to these polypeptide antigens or their derivatives was detected. These polypeptides could provide a marker for prediction or diagnosis of RA.

SEQUENCE LISTING

(1) GENERAL INFORMATION

(i) APPLICANT: KANEKA CORPORATION

(ii) TITLE OF INVENTION: AUTOANTIGEN

(iii) NUMBER OF SEQUENCE: 10

(iv) CORRESPONDENCE ADDRESS:
(A) ADDRESS: KANEKA CORPORATION
(B) STREET: 2-4, Nakanoshima 3-chome, Kita-ku
(C) CITY: Osaka-shi
(D) STATE: Osaka
(E) COUNTRY: Japan
(F) ZIP: 530

(v) COMPUTER READABLE FORM:
(1) MEDIUM TYPE: Diskette. 3.50 inch. 1.4 MB format
(2) COMPUTER: Compatible
(3) OPERATING SYSTEM: MS-DOS ver.2.11
(4) SOFTWARE: Word Perfect (ASCII file)

(vii) PRIOR APPLICATION DATA:
(A) APPLICATION NUMBER: JP 7-288957
(B) FILING DATE: 07-Nov-1995

(2) INFORMATION FOR SEQ ID NO: 1:
(i) SEQUENCE CHARACTERISTICS:
(A) LENGTH: 329 amino acids
(B) TYPE: amino acids
(D) TOPOLOGY: linear
(ii) MOLECULAR TYPE: protein
(xi) SEQUENCE DESCRIPTION: SEQ ID NO: 1:

(2) INFORMATION FOR SEQ ID NO: 2:
(i) SEQUENCE CHARACTERISTICS:
(A) LENGTH: 990 base pairs
(B) TYPE: nucleic acids
(C) STRANDEDNESS: single
(D) TOPOLOGY: linear
(ii) MOLECULAR TYPE: cDNA to mRNA
(xi) SEQUENCE DESCRIPTION: SEQ ID NO: 2:
ATGTTGACGT TGCAGACTTG GGTAGTGCAA GCCTTGTTTA TTTTCCTCAC CACTGAATCT 60
ACAGGTGAAC TTCTAGATCC ATGTGGTTAT ATCAGTCCTG AATCTCCAGT TGTACAACTT 120
CATTCTAATT TCACTGCAGT TTGTGTGCTA AAGGAAAAAT GTATGGATTA TTTTCATGTA 180
AATGCTAATT ACATTGTCTG GAAAACAAAC CATTTTACTA TTCCTAAGGA GCAATATACT 240
ATCATAAACA GAACAGCATC CAGTGTCACC TTTACAGATA TAGCTTCATT AAATATTCAG 300
CTCACTTGCA ACATTCTTAC ATTCGGACAG CTTGAACAGA ATGTTTATGG AATCACAATA 360
ATTTCAGGCT TGCCTCCAGA AAAACCTAAA AATTTGAGTT GCATTGTGAA CGAGGGGAAG 420

AAAATGAGGT GTGAGTGGGA TGGTGGAAGG GAAACACACT TGGAGACAAA CTTCACTTTA 480
AAATCTGAAT GGGCAACACA CAAGTTTGCT GATTGCAAAG CAAAACGTGA CACCCCCACC 540
TCATGCACTG TTGATTATTC TACTGTGTAT TTTGTCAACA TTGAAGTCTG GGTAGAAGCA 600
GAGAATGCCC TTGGGAAGGT TACATCAGAT CATATCAATT TTGATCCTGT ATATAAAGTG 660
AAGCCCAATC CGCCACATAA TTTATCAGTG ATCAACTCAG AGGAACTGTC TAGTATCTTA 720
AAATTGACAT GGACCAACCC AAGTATTAAG AGTGTTATAA TACTAAAATA TAACATTCAA 780
TATAGGACCA AAGATGCCTC AACTTGGAGC CAGATTCCTC CTGAAGACAC AGCATCCACC 840
CGATCTTCAT TCACTGTCCA AGACCTTAAA CCTTTTACAG AATATGTGTT TAGGATTCGC 900
TGTATGAAGG AAGATGGTAA GGGATACTGG AGTGACTGGA GTGAAGAAGC AAGTGGGATC 960
ACCTATGAAG ATAACATTGC CTCCTTTTGA 990

(2) INFORMATION FOR SEQ ID NO: 3:
(i) SEQUENCE CHARACTERISTICS:
(A) LENGTH: 308 amino acids
(B) TYPE: amino acids
(D) TOPOLOGY: linear
(ii) MOLECULAR TYPE: protein

Glu Leu Gln Lys His Gln Glu Thr Ala Glu Lys Thr Lys Arg Val Ser
290 295 300
Thr Lys Glu Ile
305

(2) INFORMATION FOR SEQ ID NO: 4:
(i) SEQUENCE CHARACTERISTICS:
(A) LENGTH: 926 base pairs
(B) TYPE: nucleic acids
(C) STRANDEDNESS: single
(D) TOPOLOGY: linear
(ii) MOLECULAR TYPE: cDNA to mRNA
(xi) SEQUENCE DESCRIPTION: SEQ ID NO: 4:
ATGTGGAAAC GCTGGCTCGC GCTCGCGCTC GCGCTGGTGG CGGTCGCCTG GGTCCGCGCC 60
GAGGAAGAGC TAAGGAGCAA ATCCAAGATC TGTGCCAATG TGTTTTGTGG AGCCGGCCGG 120
GAATGTGCAG TCACAGAGAA AGGGGAACCC ACCTGTCTCT GCATTGAGCA ATGCAAACCT 180
CACAAGAGGC CTGTGTGTGG CAGTAATGGC AAGACCTACC TCAACCACTG TGAACTGCAT 240
CGAGATGCCT GCCTCACTGG ATCCAAAATC CAGGTTGATT ACGATGGACA CTGCAAAGAG 300
AAGAAATCCG TAAGTCCATC TGCCAGCCCA GTTGTTTGCT ATCAGTCCAA CCGTGATGAG 360
CTCCGACGTC GCATCATCCA GTGGCTGGAA GCTGAGATCA TTCCAGATGG CTGGTTCTCT 420
AAAGGCAGCA ACTACAGTGA AATCCTAGAC AAGTATTTTA AGAACTTTGA TAATGGTGAT 480
TCTCGCCTGG ACTCCAGTGA ATTCCTGAAG TTTGTGGAAC AGATGAAACT GCCATCAATA 540
TTACAACGTA TCCAGACCAG GAGAACAACA AGTTGCTTAG GGGACTCTGT GTTGATGCTC 600
TCATTGAACT GTCTGATGAA AATGCTGATT GGAAACTCAG CTTCCAAGAG TTTCTCAAGT 660
GCCTCAACCC ATCTTTCAAC CCTCCTGAGA AGAAGTGTGC CCTGGAGGAT GAAACGTATG 720
CAGATGGAGC TGAGACCGAG GTGGACTGTA ACCGCTGTGT CTGTGCCTGT GGAAATTGGG 780
TCTGTACAGC CATGACCTGT GACGGAAAGA ATCAGAAGGG GGCCCAGACC CAGACAGAGG 840
AGGAGATGAC CAGATATGTC CAGGAGCTCC AAAAGCATCA GGAAACAGCT GAAAAGACCA 900
AGAGAGTGAG CACCAAAGAG ATCTAA 926

(2) INFORMATION FOR SEQ ID NO: 5:
(i) SEQUENCE CHARACTERISTICS:
(A) LENGTH: 10 amino acids
(B) TYPE: amino acids
(D) TOPOLOGY: linear
(ii) MOLECULAR TYPE: polypeptide
(xi) SEQUENCE DESCRIPTION: SEQ ID NO: 5:

Ile Thr Tyr Glu Asp Asn Ile Ala Ser Phe
1               5                   10

(2) INFORMATION FOR SEQ ID NO: 6:
(i) SEQUENCE CHARACTERISTICS:
(A) LENGTH: 15 amino acids
(B) TYPE: amino acids
(D) TOPOLOGY: linear
(ii) MOLECULAR TYPE: polypeptide
(xi) SEQUENCE DESCRIPTION: SEQ ID NO: 6 :
Glu Glu Ala Ser Gly Ile Thr Tyr Glu Asp Asn Ile Ala Ser Phe
1               5                   10                  15

(2) INFORMATION FOR SEQ ID NO: 7:
(i) SEQUENCE CHARACTERISTICS:
(A) LENGTH: 28 base pairs
(B) TYPE: nucleic acids
(C) STRANDEDNESS: single
(ii) MOLECULAR TYPE: synthetic DNA
(xi) SEQUENCE DESCRIPTION: SEQ ID NO: 7:
AAGGA TCCGA ACTTC TAGAT CCATG TGG 28

(2) INFORMATION FOR SEQ ID NO: 8:
(i) SEQUENCE CHARACTERISTICS:
(A) LENGTH: 30 base pairs
(B) TYPE: nucleic acids
(C) STRANDEDNESS: single
(ii) MOLECULAR TYPE: synthetic DNA
(xi) SEQUENCE DESCRIPTION: SEQ ID NO: 8:
TTGCG GCCGC TCAAA AGGAG GCAAT GTTAT 30

(2) INFORMATION FOR SEQ ID NO: 9:
(i) SEQUENCE CHARACTERISTICS:
(A) LENGTH: 27 base pairs
(B) TYPE: nucleic acids
(C) STRANDEDNESS: single
(ii) MOLECULAR TYPE: synthetic DNA

(xi) SEQUENCE DESCRIPTION: SEQ ID NO: 9:
AACCC GGGAG GAAGA GCTAA GGAGC AA 27

(2) INFORMATION FOR SEQ ID NO: 10:
(i) SEQUENCE CHARACTERISTICS:
(A) LENGTH: 30 base pairs
(B) TYPE: nucleic acids
(C) STRANDEDNESS: single
(ii) MOLECULAR TYPE: synthetic DNA
(xi) SEQUENCE DESCRIPTION: SEQ ID NO: 10:
TTGCG GCCGC TGTGC CTCCT CATTA GATCT 30

## Description

### TECHNICAL FIELD

The present invention relates to a novel antigenic peptide with which antibodies from rheumatoid arthritis patients specifically react, a DNA encoding the antigen, a composition for detecting antibodies specifically present in rheumatism patients through an antigen-antibody reaction using the antigen, as well as a method and a kit for detecting antibodies specifically present in rheumatism patients.

### BACKGROUND ART

Rheumatoid arthritis (RA) is a cryptogenic, chronic, progressive and intractable disease. Disease development ranges over a very long period of about 20 years on average. During this period, aggravation, remission, and exacerbation are repeated, generally resulting in privation of the limbs and the body to various degrees. The essence of RA is a chronic synovitis which does not show tendencies toward spontaneous curing, and which exhibits lymphocyte infiltration, neovascularization, stratification of synovial cells, as well as synovial cell proliferation. The persisting synovial inflammation and proliferation of inflamed tissues eventually destroy cartilage and bone, resulting in articular deformation and physical disorders. Since RA is such a long-term disease, the importance of providing adequate treatment in an early stage to prevent progression into mature rheumatism has been pointed out. As for RA diagnosis, the RA diagnosis criteria of The American Rheumatism Association [Arnett et al., Arthritis and Rheumatism 31, p.315 (1988)], revised in 1987, is widely used currently. These criteria provide clinical diagnosis methods concerning stiffness of limbs, articular swelling, and the like. Some incipient forms of RA in existence less than 1 year since its onset do not allow determination of disease types, thereby making diagnosis difficult. Therefore, there has been a desire for diagnostics which are chemically effective for identifying autoantigens and autoantibodies, etc., associated with incipient RA.

Various studies on autoantibodies in RA patients have been conducted. For example, anti-fibrillarin antibody [Kasturi et al., J. Exp. Med. 181, p.1027 (1995)], anti-RA 33 antibody [Steiner et al., J. Clin. Invest. 90, p.1061 (1992)], anti-calpastatin antibody [Mimori et al., Pro. Natl. Acad. Sci. USA 92, p.7267 (1995) and Despres et al., J. Clin. Invest. 95, p.1891 (1995)], anti-filaggrin antibody [Sebbag et al., J. Clin. Invest. 95, p.2672 (1995)], anti-annexin antibody [Yoshikata et al., J. Biol. Chem. 269, p.4240 (1994)] and the like have been identified in connection with RA. However, these are not specific to RA because they have also been identified as autoantibodies against other RAs as well as RA. Moreover, none of these are employed for diagnosis or treatment of RA. Therefore, effective diagnostics have been desired.

### DISCLOSURE OF THE INVENTION

The present invention provides a solution to the above-mentioned conventional problems, aiming at obtaining a novel antigenic polypeptide with which antibodies from RA patient specifically react; a gene encoding the antigenic polypeptide; an expression vector containing the gene; a transformant including the expression vector; providing a novel antigenic polypeptide by using the transformant; and providing a method of detection and diagnosis for RA patients using the antigenic polypeptide, as well as a composition and a kit for detection and diagnosis for RA patients using the antigenic polypeptide.

Analyzing autoantigens generated by synovial cells and specifying antigens corresponding to autoantibodies for RA facilitates diagnosis of RA, opening doors to treatment thereof. Accordingly, cDNA libraries were made by using articular synovial cells of RA patients, and a screening was conducted with respect to IgG in synovial fluid from RA patients. As a result, a clone (clone A) encoding a novel polypeptide as a rheumatism antigen, and a clone encoding follistatin-related protein (FRP), which is known as a polypeptide but novel as a rheumatism antigen, were successfully isolated.

The novel antigenic polypeptide encoded by this clone A (hereinafter referred as "clone A polypeptide") is considered as a variant of gp130 (Hibi et al., Cell 63, p.1149 (1991)), which is one of the receptors of interleukin 6 (IL-6). Gp130 is a known protein, and is a common subunit among IL-6, leukemia inhibitory factor (LIF), ciliary neurotrophic factor (CNTF), oncostatin M which is a growth factor of a certain kind of cancer cell, interleukin 11 (IL-11) and the like [Taga and Kishimoto FASEB J. 6, p.3387 (1992)], and is a protein serving the function of transmitting signals to cells. A sequence of amino acids 1 to 324 of the inventive clone A polypeptide coincides with a sequence of amino acids 1 to 324 of this gp130 (mature protein). However, 5 amino acids 325 to 329 of clone A polypeptide (i.e., 5 amino acid residues from the carboxy terminus of clone A polypeptide) were different from those of gp130. It is presumable that terminating the polypeptide with these 5 amino acids affects the receptor activation by IL6.

On the other hand, FRP is a known protein and has been cloned from a cDNA library of human Hs683 glioma. [Zwijsen et al., Eur. J. Biochem. 225, p.937 (1994)]. Due to its similarity in amino acid sequence, it is presumed to have a similar activity to that of FRP. However, its physiological function in synovial tissues has not yet been determined.

In order to advance studies on clone A polypeptide or FRP, it is necessary to obtain these polypeptides in large quantitity and with high purity. However, there is a problem in that procedures for isolating and purifying these polypeptides from synovial cells from RA patients producing clone A polypeptide or human Hs683 gliomas producing FRP [Zwijsen et al., Eur. J. Biochem. 225, p.937 (1994)] are complicated, and that only a small amount of the protein of interest can be obtained. Zwijsen et al also produced a recombinant FRP using COS1 cells. However, a method for obtaining these polypeptides with high purity and in a large amount has been desired for availing it as a diagnostic according to the present invention. The inventors of the present invention allowed clone A polypeptide and FRP to express as glutathione S-transferase (GST) fusion protein, thereby succeeding in obtaining a large amount of purified polypeptide. As a result of expressing clone A polypeptide and FRP in Escherichia coli and examining its reactivity with sera from RA patients, it webs found that FRP reacts specifically with sera from RA patients.

Moreover, in clone A polypeptide, it was predicted that a polypeptide containing 5 amino acids at the carboxy terminus, which is a mutated part of the polypeptide, may serve as a B cell antigen. A polypeptide (C10 polypeptide) consisting of 10 amino acids from the carboxy terminus including these 5 amino acids and a polypeptide (C15 polypeptide) consisting of 15 amino acids from the carboxy terminus including these 5 amino acids were chemically synthesized, and an ELISA system was constructed employing them as antigens. Antibodies to these antigens were then examined with sera from RA patients and those from other autoimmune disease patients to reveal the surprising fact that they react specifically with the sera from RA patients.

Accordingly, it was found that detection of antibodies against these antigens could provide a marker for prediction or diagnosis of RA, and hence the present invention was accomplished.

The present invention relates to a polypeptide shown in SEQ ID No: 5 of the Sequence Listing, a polypeptide including the amino acid sequence of SEQ ID NO: 5, or a polypeptide including a substitution, deletion or addition in at least one amino acid in the amino acid sequence of SEQ ID NO: 5, the polypeptide binding to an antibody which is specific to RA patients.

Furthermore, the present invention relates to a polypeptide shown in SEQ ID NO: 6 of the Sequence Listing, a polypeptide including the amino acid sequence of SEQ ID NO: 6, or a polypeptide including a substitution, deletion or addition in at least one amino acid in the amino acid sequence of SEQ ID NO: 6, the polypeptide binding to an antibody which is specific to RA patients.

Furthermore, the present invention relates to a polypeptide including a portion of the amino acid sequence shown in SEQ ID NO: 1 of the Sequence Listing, the polypeptide binding to an antibody which is specific to RA patients.

The present invention also relates to a polypeptide including the amino acid sequence shown in SEQ ID NO: 1 of the Sequence Listing, the polypeptide binding to an antibody which is specific to RA patients.

Furthermore, the present invention relates to a polypeptide having the amino acid sequence shown in SEQ ID No: 1 of the Sequence Listing, or a polypeptide including a substitution, deletion or addition in at least one amino acid in the amino acid sequence, the polypeptide binding to an antibody which is specific to RA patients.

Furthermore, the present invention relates to a polypeptide including a substitution, deletion or addition in at least one amino acid in the amino acid sequence shown in SEQ ID No: 3 of the Sequence Listing, the polypeptide binding to an antibody which is specific to RA patients.

The present invention also relates to a DNA encoding a polypeptide binding to an antibody which is specific to RA patients.

In a preferred embodiment, the DNA is a DNA encoding any one of the following polypeptides:
(a) a polypeptide shown in SEQ ID NO: 5 of the Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to RA patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to RA patients;
(g) a polypeptide having the amino acid sequence shown in SEQ ID NO: 1 of the Sequence Listing, the polypeptide binding to an antibody which is specific to RA patients;
(h) a polypeptide which is a fragment of the amino acid sequence shown in SEQ ID NO: 1 of the Sequence Listing, the polypeptide binding to an antibody which is specific to RA patients;
(i) a polypeptide including the amino acid sequence shown in SEQ ID NO: 1 of the Sequence Listing, the polypeptide binding to an antibody which is specific to RA patients;
(j) a polypeptide including a substitution, deletion or addition in at least one amino acid in the amino acid sequence shown in SEQ ID NO: 1 of the Sequence Listing, the polypeptide binding to an antibody which is specific to RA patients;
(k) a polypeptide which is a fragment of the amino acid sequence shown in SEQ ID NO: 3 of the Sequence Listing, the polypeptide binding to an antibody which is specific to RA patients;
(l) a polypeptide including the amino acid sequence shown in SEQ ID NO: 3 of the Sequence Listing, the polypeptide binding to an antibody which is specific to RA patients; and
(m) a polypeptide including a substitution, deletion or addition in at least one amino acid in the amino acid sequence shown in SEQ ID NO: 3 of the Sequence Listing, the polypeptide binding to an antibody which is specific to RA patients.

In a preferred embodiment, the DNA is a DNA shown in SEQ ID NO: 2 or SEQ ID NO: 4 of the Sequence Listing.

The present invention also relates to an expression vector including a DNA encoding a polypeptide binding to an antibody which is specific to RA patients.

In a preferred embodiment, the present invention relates to an expression vector including a DNA encoding any one of the following polypeptides:
(a) a polypeptide shown in SEQ ID NO: 5 of the Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to RA patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to RA patients;
(g) a polypeptide having the amino acid sequence shown in SEQ ID NO: 1 of the Sequence Listing, the polypeptide binding to an antibody which is specific to RA patients;
(h) a polypeptide which is a fragment of the amino acid sequence shown in SEQ ID NO: 1 of the Sequence Listing, the polypeptide binding to an antibody which is specific to RA patients;
(i) a polypeptide including the amino acid sequence shown in SEQ ID NO: 1 of the Sequence Listing, the polypeptide binding to an antibody which is specific to RA patients;
(j) a polypeptide including a substitution, deletion or addition in at least one amino acid in the amino acid sequence shown in SEQ ID NO: 1 of the Sequence Listing, the polypeptide binding to an antibody which is specific to RA patients;
(k) a polypeptide which is a fragment of the amino acid sequence shown in SEQ ID NO: 3 of the Sequence Listing, the polypeptide binding to an antibody which is specific to RA patients;
(l) a polypeptide including the amino acid sequence shown in SEQ ID NO: 3 of the Sequence Listing, the polypeptide binding to an antibody which is specific to RA patients; and
(m) a polypeptide including a substitution, deletion or addition in at least one amino acid in the amino acid sequence shown in SEQ ID NO: 3 of the Sequence Listing, the polypeptide binding to an antibody which is specific to RA patients.

The present invention also relates to a transformed cell including the above-mentioned expression vector.

Moreover, the present invention relates to a composition for detecting an antibody which is specific to RA patients, including a polypeptide binding to an antibody which is specific to RA patients.

In a preferred embodiment, the composition is a composition containing at least one polypeptide selected from a group consisting of:
(a) a polypeptide shown in SEQ ID NO: 5 of the Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to RA patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to RA patients;
(g) a polypeptide having the amino acid sequence shown in SEQ ID NO: 1 of the Sequence Listing, the polypeptide binding to an antibody which is specific to RA patients;
(h) a polypeptide which is a fragment of the amino acid sequence shown in SEQ ID NO: 1 of the Sequence Listing, the polypeptide binding to an antibody which is specific to RA patients;
(i) a polypeptide including the amino acid sequence shown in SEQ ID NO: 1 of the Sequence Listing, the polypeptide binding to an antibody which is specific to RA patients;
(j) a polypeptide including a substitution, deletion or addition in at least one amino acid in the amino acid sequence shown in SEQ ID NO: 1 of the Sequence Listing, the polypeptide binding to an antibody which is specific to RA patients;
(k) a polypeptide which is a fragment of the amino acid sequence shown in SEQ ID NO: 3 of the Sequence Listing, the polypeptide binding to an antibody which is specific to RA patients;
(l) a polypeptide including the amino acid sequence shown in SEQ ID NO: 3 of the Sequence Listing, the polypeptide binding to an antibody which is specific to RA patients;
(m) a polypeptide including a substitution, deletion or addition in at least one amino acid in the amino acid sequence shown in SEQ ID NO: 3 of the Sequence Listing, the polypeptide binding to an antibody which is specific to RA patients; and
(n) a polypeptide having the amino acid sequence shown in SEQ ID NO: 3 of the Sequence Listing, the polypeptide binding to an antibody which is specific to RA patients.

In a preferred embodiment, the polypeptide is a polypeptide obtained by culturing:
(1) a transformed cell transformed with the above-mentioned expression vector;
(2) a transformed cell including an expression vector including a DNA which encodes a polypeptide having the amino acid sequence shown in SEQ ID NO: 3 of the Sequence Listing and binding to an antibody which is specific to RA patients; or
(3) a transformed cell including an expression vector including a DNA shown in SEQ ID NO: 4 of the Sequence Listing.

Moreover, the present invention is a method for detecting an antibody which is specific to RA patients including the steps of: reacting a test sample with a polypeptide binding specifically to an antibody which is specific to RA patients, and detecting the reaction product.

In a preferred embodiment, the polypeptide is at least one polypeptide selected from a group consisting of:
(a) a polypeptide shown in SEQ ID NO: 5 of the Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to RA patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to RA patients;
(g) a polypeptide having the amino acid sequence shown in SEQ ID NO: 1 of the Sequence Listing, the polypeptide binding to an antibody which is specific to RA patients;
(h) a polypeptide which is a fragment of the amino acid sequence shown in SEQ ID NO: 1 of the Sequence Listing, the polypeptide binding to an antibody which is specific to RA patients;
(i) a polypeptide including the amino acid sequence shown in SEQ ID NO: 1 of the Sequence Listing, the polypeptide binding to an antibody which is specific to RA patients;
(j) a polypeptide including a substitution, deletion or addition in at least one amino acid in the amino acid sequence shown in SEQ ID NO: 1 of the Sequence Listing, the polypeptide binding to an antibody which is specific to RA patients;
(k) a polypeptide which is a fragment of the amino acid sequence shown in SEQ ID NO: 3 of the Sequence Listing, the polypeptide binding to an antibody which is specific to RA patients;
(l) a polypeptide including the amino acid sequence shown in SEQ ID NO: 3 of the Sequence Listing, the polypeptide binding to an antibody which is specific to RA patients;
(m) a polypeptide including a substitution, deletion or addition in at least one amino acid in the amino acid sequence shown in SEQ ID NO: 3 of the Sequence Listing, the polypeptide binding to an antibody which is specific to RA patients; and
(n) a polypeptide having the amino acid sequence shown in SEQ ID NO: 3 of the Sequence Listing, the polypeptide binding to an antibody which is specific to RA patients.

Furthermore, the present invention relates to a kit for detecting an antibody which is specific to RA patients, the kit including a polypeptide binding specifically to an antibody which is specific to RA patients.

In a preferred embodiment, the present invention relates to a kit wherein the polypeptide is at least one polypeptide selected from a group consisting of:
(a) a polypeptide shown in SEQ ID NO: 5 of the Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to RA patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to RA patients;
(g) a polypeptide having the amino acid sequence shown in SEQ ID NO: 1 of the Sequence Listing, the polypeptide binding to an antibody which is specific to RA patients;
(h) a polypeptide which is a fragment of the amino acid sequence shown in SEQ ID NO: 1 of the Sequence Listing, the polypeptide binding to an antibody which is specific to RA patients;
(i) a polypeptide including the amino acid sequence shown in SEQ ID NO: 1 of the Sequence Listing, the polypeptide binding to an antibody which is specific to RA patients;
(j) a polypeptide including a substitution, deletion or addition in at least one amino acid in the amino acid sequence shown in SEQ ID NO: 1 of the Sequence Listing, the polypeptide binding to an antibody which is specific to RA patients;
(k) a polypeptide which is a fragment of the amino acid sequence shown in SEQ ID NO: 3 of the Sequence Listing, the polypeptide binding to an antibody which is specific to RA patients;
(l) a polypeptide including the amino acid sequence shown in SEQ ID NO: 3 of the Sequence Listing, the polypeptide binding to an antibody which is specific to RA patients;
(m) a polypeptide including a substitution, deletion or addition in at least one amino acid in the amino acid sequence shown in SEQ ID NO: 3 of the Sequence Listing, the polypeptide binding to an antibody which is specific to RA patients; and
(n) a polypeptide having the amino acid sequence shown in SEQ ID NO: 3 of the Sequence Listing, the polypeptide binding to an antibody which is specific to RA patients.

In a preferred embodiment, the polypeptide is a polypeptide obtained by culturing:
(1) the above-mentioned transformed cell;
(2) a transformed cell including an expression vector including a DNA which encodes a polypeptide having the amino acid sequence shown in SEQ ID NO: 3 of the Sequence Listing and binding to an antibody which is specific to RA patients; or
(3) a transformed cell including an expression vector including a DNA shown in SEQ ID NO: 4 of the Sequence Listing.

### BRIEF DESCRIPTION OF THE DRAWINGS

Fig.1 is a figure showing results of western blotting using IgG of synovial fluid from RA patients as a probe and using clone A polypeptide, which is a novel protein of the present invention, as an antigen.

Fig.2 is a figure showing results of western blotting using IgG of synovial fluid from RA patients as a probe and using FRP as an antigen.

Fig .3 is a figure showing cloning sites of clone A and FRP.

Fig.4 is a figure showing results of western blotting for recombinant clone A polypeptide and FRP.

Fig.5 is a figure showing HPLC patterns of a chemically synthesized C10 polypeptide and a chemically synthesized C15 polypeptide using an ODS column.

Fig.6 is a figure showing standard curves of ELISA systems using C10 polypeptide and C15 polypeptide as antigens.

Fig.7 is a scatter diagram showing results of anti-C10 polypeptide antibody measurement in rheumatism related diseases.

### BEST MODES FOR CARRYING OUT THE INVENTION

A polypeptide used in the present invention includes polypeptides respectively having the amino acid sequences shown in SEQ ID NO: 1, SEQ ID NO: 3, SEQ ID NO: 5, and SEQ ID NO: 6, as well as derivatives thereof. Specifically, the following polypeptides (a) to (n) are included:
(a) a polypeptide shown in SEQ ID NO: 5 of the Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to RA patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to RA patients;
(g) a polypeptide having the amino acid sequence shown in SEQ ID NO: 1 of the Sequence Listing, the polypeptide binding to an antibody which is specific to RA patients;
(h) a polypeptide which is a fragment of the amino acid sequence shown in SEQ ID NO: 1 of the Sequence Listing, the polypeptide binding to an antibody which is specific to RA patients;
(i) a polypeptide including the amino acid sequence shown in SEQ ID NO: 1 of the Sequence Listing, the polypeptide binding to an antibody which is specific to RA patients;
(j) a polypeptide including a substitution, deletion or addition in at least one amino acid in the amino acid sequence shown in SEQ ID NO: 1 of the Sequence Listing, the polypeptide binding to an antibody which is specific to RA patients;
(k) a polypeptide which is a fragment of the amino acid sequence shown in SEQ ID NO: 3 of the Sequence Listing, the polypeptide binding to an antibody which is specific to RA patients;
(l) a polypeptide including the amino acid sequence shown in SEQ ID NO: 3 of the Sequence Listing, the polypeptide binding to an antibody which is specific to RA patients;
(m) a polypeptide including a substitution, deletion or addition in at least one amino acid in the amino acid sequence shown in SEQ ID NO: 3 of the Sequence Listing, the polypeptide binding to an antibody which is specific to RA patients; and
(n) a polypeptide having the amino acid sequence shown in SEQ ID NO: 3 of the Sequence Listing, the polypeptide binding to an antibody which is specific to RA patients.

In the present invention, the polypeptide shown in SEQ ID NO: 1 is referred as clone A polypeptide, whereas the polypeptide shown in SEQ ID NO: 3 is referred as FRP. A polypeptide which is a fragment of clone A polypeptide shown in SEQ ID NO: 5 consisting of 10 amino acids from the carboxy terminus of clone A polypeptide is referred as C10 polypeptide. A polypeptide which is a fragment of clone A polypeptide shown in SEQ ID NO: 6 consisting of 15 amino acids from the carboxy terminus of clone A polypeptide is referred as C15 polypeptide.

The "derivatives thereof" include a polypeptide including the amino acid sequence shown in SEQ ID NO: 1, SEC ID NO: 3, SEQ ID NO: 5 or SEQ ID NO: 6, or a polypeptide including a substitution, deletion or addition in at least one amino acid of the amino acid sequence of SEQ ID NO: 1, SEQ ID NO: 3, SEQ ID NO: 5 or SEQ ID NO: 6, the polypeptides binding to an antibody which is specific to RA patients. The "derivatives thereof" also include a polypeptide including a portion of the amino acid sequence (occasionally referred to as a "fragment of a polypeptide") shown in SEQ ID NO: 1 or SEQ ID NO: 3 of the Sequence Listing and binding to an antibody which is specific to RA Patients.

The term "polypeptide" refers to a compound including a plurality of amino acids combined.

A polypeptide according to the present invention is obtained by chemical synthesis or by culturing a transformed cell which is transformed by an expression vector described later. A fragment of a polypeptide can also be produced by using an appropriate proteolytic enzyme. It can be determined whether or not the obtained polypeptide or fragment reacts with an antibody through reactions with sera obtained from RA patients. This method is well-known to those skilled in the art, and the same method as the method for detecting antibodies described later can be applied.

C10 polypeptide (SEQ ID NO: 5) and C15 polypeptide (SEQ ID NO: 6) of the present invention is obtained by a known chemical synthesis such as solid phase synthesis or liquid phase synthesis [for example, see "SHIN SEIKAGAKU JIKKEN KOUZA Vol.1, TANPAKUSHITSU VI GOUSEI OYOBI HATSUGEN" (Synthesis and Expression, Protein VI, vol.1 New biochemical Experiment Lectureship), TOKYO KAGAKU DOUJIN K.K., published in 1992, pp.3-66]. The obtained peptide is purified by chromatography by a reverse phase ODS, hydrophobic chromatography or ion exchange chromatography to give the peptide of interest.

A polypeptide in which at least one amino acid is deleted, substituted or added, can be produced, for example, based on the gene sequence of clone A or FRP by altering the gene sequence with a well-known method, for example, site-directed mutagenesis or deletion mutagenesis using M13 phage.

A polypeptide according to the present invention can also be produced as a fusion protein with another polypeptide. For example, the polypeptide can be expressed as a fusion protein with superoxide dismutase (SOD), thioredoxin (TRX), or glutathione-S-transferase (GST). When using GST, the fusion protein is cleaved with thrombin after culture so as to release the protein of interest from GST, thereby obtaining the protein of interest. A kit using this method is sold by and available from Pharmacia and the like.

A DNA encoding a polypeptide binding to an antibody which is specific to RA patients or its derivatives according to the present invention can be chemically synthesized by a method well-known to those skilled in the art, or can be obtained from a cDNA library of synovial cells by a genetic engineering method (e.g., hybridization or PCR), based on the sequence disclosed herein.

As for DNAs according to the present invention, DNAs encoding the above-mentioned polypeptide sequences can be suitably used.

Expression vectors according to the present invention include DNAs encoding the above-mentioned polypeptides. Examples of expression vectors include plasmids; viruses; or phage vectors including an ori region and, if necessary, a promoter for the expression of the above-mentioned DNA, a control element for the promotor, and the like. The vector can contain one or more selectable marker gene, for example, ampicillin resistance gene or the like. The method for integrating a gene which expresses the polypeptide of interest into such a plasmid or vector is well-known to those skilled in the art.

An expression vector for a procaryote, e.g., Escherichia coli, can be constructed by connecting, downstream of an appropriate promoter, DNA including an initiation codon (ATG) at the 5' terminus of a DNA encoding a mature protein portion as well as a translation termination codon (TAA, TGA or TAG) at the 3' terminus, and then inserting this into a vector which functions in Escherichia coli. Examples of promoters include tac promoter, trp promoter, lac promoter, T7 promoter and the like; however, any appropriate promoter can be used depending on the host to be used for expressing the gene. As for the vector, a vector which includes a marker gene which can confer selectivity of phenotypes to a transformed cell is preferable. Examples of such vectors include but are not limited to pET system vector, pExCell, pBR322, pUC18, pUC19 and the like. Escherichia coli which has been transformed by such an expression vector may be cultured in an appropriate medium to express a polypeptide of interest in the bacterial body.

When the host is an eucaryotic cell, a promoter and/or a RNA splicing site is added upstream of the gene to be expressed, and a polyadenylation signal or the like is added downstream of the gene, and these are inserted into an expression vector corresponding to the host. As for this vector, a vector containing an origin of replication, a selectable marker, and the like, is desirable. In the case of a yeast, alcohol dehydrogenase (ADH) promoter, CYC promoter, PhoA promoter or the like from Saccharomyces cerevisiae can be used as a promoter for use in the expression vector. Expression vectors (pHIL-D2, pPIC9 and the like) using alcohol oxidase AOXI promoter from Pichia pastris are sold by and available from Invitrogen BV. As for a drug resistance marker, G418 resistance gene can be used.

Examples of promoters for gene expression in animal cells include but are not limited to SV40 promoter, LTR promoter, metallothionein promoter or the like. Examples of expression vectors include but are not limited to retrovirus vectors, vaccinia virus vector, papilloma virus vector, SV40-derived vectors, and the like.

In the translation stage from a DNA to a polypeptide, since 1 to 6 kinds of codons encoding one amino acid are known (1 kind for Met, 6 kinds for Leu), it is possible to change the DNA nucleotide sequence without changing the amino acid sequence of the polypeptide. By changing the nucleotide sequence, the productivity of the polypeptide may often be improved.

A vector can be used for the production of an RNA corresponding to a DNA, as well as the transformation of a host cell. An antisense RNA can also be produced by inserting a DNA to an antisense region of a vector. Such an antisense RNA also can be used to regulate the polypeptide level in the cell.

A transformant according to the present invention can be obtained by introducing the above-mentioned expression vector into a host cell by a method well-known to those skilled in the art. As for host cells, for example, procaryotes such as bacteria, or eucaryotes such as yeasts, insect cells, mammalian cells or plant cells can be used.

As for bacteria, Escherichia coli, Bacillus subtilis (Bacilli), or the like can be suitably used without limitation. Examples of Escherichia coli include JM109, HB101, DH5α and the like.

As for yeasts, Saccharomyces cerevisiae, Pichia pastris or the like can be suitably used.

As for animal cells, those which are established as cell lines are preferable. For example, COS cells, CHO cells, 3T3 cells, Hela cells, human FL cells or the like can be suitably used without limitation.

By culturing such transformants, a polypeptide according to the present invention can be expressed and collected. The culture can be carried out under conditions which allow the polypeptide to be expressed and produced from the host cells.

For the purification of polypeptides, a known method, for example, chromatography such as gel filtration chromatography, ion exchange chromatography, affinity chromatography, reverse phase liquid chromatography can be used alone or in combination to effect purification.

A composition according to the present invention for detecting an antibody which is specific to RA patients including a peptide binding to an antibody which is specific to RA patients contains at least one peptide selected from the group consisting of the above-mentioned (a) to (n) polypeptides. Preferably, a composition according to the present invention contains clone A polypeptide, FRP polypeptide or fragments thereof (e.g., C10 polypeptide and C15 polypeptide).

A composition according to the present invention can be used in a method or a kit for detecting or diagnosing to an antibody which is specific to RA patients.

A method for detecting or diagnosing an antibody which is specific to RA patients includes the steps of: reacting a test sample with a polypeptide binding specifically to an antibody which is specific to RA patients, and detecting a reaction product.

"Antibody" refers to a component existing in body fluids of RA patients that is raised by a particular antigenic substance. Examples of antibodies in RA patients include IgM, IgG, IgE, IgD, IgA, and the like.

The reaction between the polypeptide (antigen) according to the present invention and antibodies is carried out under conditions well-known to those skilled in the art. The conditions well-known to those skilled in the art are applied as conditions for reacting an antigen with an antibody. A method which is known to those skilled in the art can be applied as a method for detecting an antigen-antibody reaction product. Examples of detection methods include the precipitation reaction method, the ELISA method, the RIA method, the western blotting method, and the like. For example, appropriately diluted sera from patients are allowed to react with the antigen, followed by a washing, and thereafter a reaction is carried out by adding anti-human IgG antibodies which are labeled with peroxidase as secondary antibodies. Thereafter ABTS, which is a substrate of peroxidase, is added for coloration development, and thus antibodies can be detected by measuring the absorbance at 415 nm.

A kit according to the present invention for detecting an antibody which is specific to RA patients can contain further components which are capable of detecting the resultant antigen-antibody complex. These components are, for example, components which are applicable to methods such as the precipitation reaction method, the ELISA method, the RIA method, and the western blotting method.

A kit according to the present invention can contain an ELISA plate on which a polypeptide according to the present invention, e.g., clone A polypeptide, C10 polypeptide, C15 polypeptide, FRP polypeptide or derivatives thereof are immobilized, and reagents for detecting an antigen-antibody complex resulting from binding to antibodies from RA patients. In addition, appropriate reagents depending on the measuring method, e.g., coloring development reagents, reaction stopping reagents, standard antigen reagents, or reagents for pre-treatment of samples, can be appropriately selected and attached to the kit as necessary.

The reagent for the detection can contain a component which is applicable to methods such as the precipitation reaction method, the ELISA method, the RIA method or the western blotting method. In the case of the ELISA method, a secondary antibody reagent can be used as a reagent for the detection, for example. A secondary antibody reagent is goat or murine anti-human IgG or anti-human (IgA+IgG+IgM) that reacts with human IgG, IgM, and IgA. These secondary antibodies can be labeled with a labeling agent for use in immunoassays in general. Examples of such labeling agents to be used include radioisotopes (e.g., ³²P, ³H, ¹²⁵I), enzymes (e.g., β-galactosidase, peroxidase, alkaline phosphatase, glucose oxidase, lactate oxidase, alcohol oxidase, or monoamine oxidase), coenzymes, prosthetic groups (e.g., FAD, FMN, ATP, biotin, hem), fluorescein derivatives (e.g., fluorescein isothiocyanate or fluorescein thiofurbamyl), rhodamine derivatives (e.g., tetramethylrhodamine B isothiocyanate), umbelliferone and 1-anilino-8-naphthalene sulfonic acid, luminol derivatives (e.g., luminol or isoluminol). Preferably, without limitation, alkaline phosphatase or peroxidase is used. In the former case, paranitrophenylphosphoric acid or the like can be used as a substrate, and in the latter case, 2,2'-azino-di-(3-ethylbenzthiazoline)-6-sulfonic acid (ABTS), and orthophenylenediamine (OPD) can be used as a substrate, although no limitation is intended.

Binding between antibodies and labeling agents can be carried out by a method appropriately selected from among known methods, e.g., that described in the publication [e.g., "ZOKU SEIKAGAKU JIKKEN KOUZA 5; MEN-EKI SEIKAGAKU KENKYUHOU" (Further Biochemistry Experiment Lectureship 5; Immunobiochemistry studying method), TOKYO KAGAKU DOUJIN K.K., published in 1986, pp.102-112]. Many of the labeled secondary antibodies are commercially sold and available. For example, peroxidase-labeled goat anti-human IgG antibody can be purchased from Cappel.

Example forms of the kit include a form in which the antigen is contained in an appropriate carrier such as a container, a resin, a membrane or a film; and a form in which the antigen is fixed on a carrier such as a container, a resin, a membrane or a film.

Examples of carriers for polypeptides include synthesized organic polymer compounds such as polyvinyl chloride, polystyrene, styrene-divinylbenzene copolymer, styrene-maleic acid anhydride copolymer, nylon, polyvinyl alcohol, polyacrylamide, polyacrylonitrile, polypropylene, and polymethylene methacrylate; polysaccharides such as dextran derivatives (e.g., Sephadex), agarose gel (e.g., Sepharose, Bio-Gel), cellulose (e.g., paper disk, filter paper); and inorganic polymer compounds such as glass, silica gel, and silicone. These compounds can be chemical compounds to which functional group such as amino group, carboxyl group, carbonyl group, hydroxyl group, sulfhydryl group or the like is introduced. Among these examples, polystyrene and polyvinyl chloride are especially preferable.

The carrier can be in any form, such as plates (e.g., microtiter plates or disks), particles (e.g., beads), tubes (e.g., test tubes), fibers, membranes, microparticles (e.g., latex particles), capsules, vacuoles and a carrier of a preferable form can be appropriately selected depending on the measuring method. Preferably the carrier is a 96-well microtiter plate capable of processing a large number of samples at one time in an ELISA system, e.g., EB plate (manufactured by Labsystems Oy), H type plates, C type plates (manufactured by Sumitomo Bakelite Co., Ltd.), Maxisorb plates (manufactured by Nunc), E.I.A./R.I.A. plates (manufactured by Costar).

The binding between a carrier and a polypeptide (antigen) can be effected by a known method such as the physical adsorption method, the ionic bond method, the covalent bond method, the conjugate method [see for example "KOTEIKA KOSO" (Immobilized Enzyme) edited by Chibata Ichirou, March 20, 1975, Kodansya K. K.; Wong, S.S. Chemistry of Protein Conjugation and Crosslinking (1991) CRC press, Inc. or Butler, J. E. Immunochemistry of Solid-Phase Immunoassay (1991) CRC press, Inc.]. Particularly preferable is the physical adsorption method because it provides for simplicity in the case of polypeptides.

Since C10 polypeptide and C15 polypeptide according to the present invention are low molecular weight polypeptides, it is presumable that physical adsorption of the polypeptide to a carrier hardly occurs. Accordingly, direct immobilization through covalent bonds can be adopted by using a carrier in which the above-mentioned functional groups are introduced and a cross-linking agent, etc., or the binding can be effected via a further substance (a spacer or a carrier) and the like between the polypeptide and the carrier. Various carrier proteins are contemplated for binding to low molecular weight polypeptides. Examples include, without limitation, BSA [Shirahama et al., Colloid Polym. Sci. 263, p.141 (1985), Alcian Blue [Jacqueline et al., J. Immunol. Methods 175, p.131 (1994)] or polylysine [Ball et al., J. Immunol. Methods 171, p.37 (1994)]. A carrier protein which exhibits little non-specific binding in tested sera can be appropriately selected. Binding between a carrier protein and a peptide can be effected by a method which utilizes covalent bonds with a cross-linking agent [e.g., glutaraldehyde, m-maleimidebenzoyl-N-hydroxysuccinimide ester], a method utilizing disulfide bonds between cysteine residues existing (or introduced) in both the carrier protein and the peptide, a method utilizing bonds between biotin and avidin respectively introduced in both substances (see the above-mentioned publication), or the like without limitation. As an assay plate suitable for these methods, for example, amine-bound plates (manufactured by Costar), carbohydrate-bound plates (manufactured by Costar), sulfhydryl-bound plates (manufactured by Costar), Amino Plates (manufactured by Sumitomo Bakelite Co., Ltd.), Carbo Plates (manufactured by Sumitomo Bakelite Co., Ltd.) and the like are commercially sold and available. The present invention illustrates examples where, the C10 polypeptide or C15 polypeptide is allowed to be bound to BSA by using glutaraldehyde, and thereafter an ELISA system is constructed by effecting binding by physical adsorption to the carrier.

The immobilized antigen can be subjected to a blocking treatment using a blocking agent, e.g., gelatin or BSA, in order to suppress nonspecific binding.

By using a thus-prepared antigen, an antibody which is specific to RA patients can be detected for use in diagnosis.

Hereinafter, a method for screening out antigens reacting with an antibody from RA patients and for specifying the antigen as a novel protein (clone A polypeptide) or FRP polypeptide, a method for producing such proteins, and a method for measuring anti-C10 antibody and anti-FRP antibody in an ELISA system or western blotting using C10 polypeptide or C15 polypeptide consisting of the carboxy terminal portion of the novel protein or FRP polypeptide are described.

It can be confirmed that a RA patient has a specific autoantibody against synovial cells by, for example, a western blotting method using IgG in body fluids, preferably articular synovial fluid, of the RA patient as a probe and using articular synovial cells from the RA patient as an antigen [Sambrook et al., Molecular Cloning, Cold Spring Harbor Laboratory, 18. 60 (1989)]. A synovial tissue obtained on an articular synovectomy for a RA patient is digested with an appropriate enzyme for cell separation, e.g., collagenase, thereby separating cells, followed by a culture for several weeks to remove suspended cells. The resultant adherent cells can be used for various purposes as synovial cells. Herein, firstly, these synovial cells are treated to give synovial cell lysate, and western blotting is carried out. For example, synovial cells on the order of 100,000 to 500,000 are dissolved in a sample buffer containing 2-mercaptethanol and SDS, and boiled for 5 to 10 minutes. After rapidly cooling the sample on ice, SDS-polyacrylamide gel electrophoresis (SDS-PAGE) is carried out. After the electrophoresis, the protein band is transferred onto a nylon membrane according to a usual method, and non-specific binding is blocked by using skim milk or the like. On the other hand, synovial fluid is sampled from an articulation of a RA patient. Cells are removed by centrifugation to leave only the liquid components, and IgG is purified on a column. As for the column, a protein A column, e.g., Prosep A (manufactured by BoxyBrown) can be used. This purified IgG fraction and the synovial cell lysate transferred onto the above-mentioned nylon membrane are allowed to react. After washing the nylon membrane, a further reaction is carried out by using an appropriate detection reagent, e.g., peroxidase-conjugated anti-human IgG antibody. After washing, the presence of an antigen can be determined by detecting the band through chemiluminescence effected with a detection agent, e.g., an ECL kit (manufactured by Amersham).

Preparation of cDNA including the nucleotide sequences shown in SEQ ID NO: 2 or SEQ ID NO: 4 of the Sequence Listing can be carried out in the following steps: (i) mRNA is separated from cells producing the autoantigen according to the present invention, e.g., articular synovial cell from RA patients; (ii) a single stranded cDNA, and subsequently a double stranded cDNA, are synthesized from the mRNA (synthesis of cDNA); (iii) the cDNA is integrated into an appropriate phage vector; (iv) the resultant recombinant phage is packaged and allowed to infect a host cell, thereby amplifying the cDNA library (preparation of the cDNA library); (v) screening is repeated for this cDNA library by using IgG purified from a body fluid from RA patients, e.g., articular synovial fluid, as a probe to obtain a single clone; and (vi) a plasmid is prepared from the resultant phage single clone, and the cDNA sequence and putative amino acid sequence can be determined by sequencing the clone.

These steps are described in more detail: In step (i), RNA is collected from the above-mentioned cultured synovial cells and mRNA including poly A can be further purified with resin. As for the resin for purification, for example, Oligo-dT bound latex resin (commercially available from Takara Co., Ltd.) or the like can be used.

Steps (ii) and (iii) are steps for producing a cDNA library and can be carried out by modifying the method by Gubler and Hoffman [Gene 25, p.263 (1983)]. These steps can also be carried out by using a combination of commercially available cDNA synthesis kits and reagents, e.g., TimeSaver cDNA Synthesis Kit (manufactured by Pharmacia), reverse transcriptase (manufactured by Stratagene), restriction enzymes (manufactured by Takara Co., Ltd.), and the like.

As for the phage vector for use in step (iii), many are known that function in Escherichia coli (e.g., λgt10, λgt11, λExCell). Preferably, λExCell (manufactured by Pharmacia), which functions in Escherichia coli, is used.

As for the packaging in step (iv), packaging a phage DNA into a coat protein of phage λ enables the phage to infect Escherichia coli and proliferate therein. A commercially available packaging kit or the like can be used in this step; e.g., Gigapack II packaging kit (manufactured by Stratagene) is preferable. Many are known as Escherichia coli for use in infection; preferably, the NM522 strain of Escherichia coli attached to packaging kits is used. The amplification of the library can be carried out by various known methods [e.g., Sambrook et al., Molecular Cloning, 8.78 (1989)].

In step (v), screening is carried out by using IgG purified from the above-mentioned articular synovial fluid as a probe. The anti-Escherichia coli antibodies can be removed from the purified IgG by, for example, treatment with lysate of the above-mentioned host of Escherichia coli NM522 strain [Smbrookr et al., Molecular Cloning, 12.26 (1989)]. As a result, the background noise during the screening can be lowered and the screening efficiency can be improved. Screening and cloning can be carried out by known methods [Sambrook et al., Molecular Cloning, 12.11 (1989)]. For example, a cDNA phage library prepared in the above-mentioned step (iv) is plated and cultured at 37°C so as to allow phage plaques to emerge. Next, a nitrocellulose membrane (manufactured by Waters), for example, is placed over the plaques. Concurrently with inducing protein synthesis, the phage is transferred onto the membrane. After washing the membrane onto which the phage is transferred with phosphate-buffered saline (PBS), blocking is carried out by using 5% skim milk (manufactured by DIFCO)/PBS and the like. Moreover, after washing with PBS, a reaction is carried out by adding purified synovial IgG which has absorbed the above-mentioned anti-Escherichia coli antibodies. By treating this membrane after transfer with an anti-human IgG antibody labeled with, e.g., horseradish peroxidase (HRP), positive phages binding to IgG can be detected. Thus, positive phage clones are picked up in a screening using IgG in synovial fluid from RA patients, and such cloning is repeated at least 3 times to finally obtain 100% positive phage clones.

Step (vi) can be carried out, e.g., according to the instructions attached to λExCell cloning vector (manufactured by Pharmacia). Specifically, a phage vector is converted into a plasmid by an in vitro excision in Escherichia coli NM522 strain as a host. After culturing this Escherichia coli, the plasmid is prepared from this Escherichia coli, and a more stable plasmid-expressing strain can be obtained by transforming another strain of Escherichia coli, e.g., DH5α strain. From this strain, a plasmid is prepared for sequencing. The sequencing is carried out by various known methods, e.g., the dideoxy-terminator method.

By a method including the above-mentioned steps (i) to (vi), two kinds of clones, including either clone A polypeptide (which is a novel polypeptide according to the present invention) or a known FRP, are isolated, indicative that these polypeptides are autoantigens associated with RA.

After an antigenic polypeptide is expressed as a fusion protein with GST, the polypeptide of interest can be obtained by being cut off the GST portion. Hereinafter, a method using Glutathione S-transferase (GST) Gene Fusion System (manufactured by Pharmacia), which is an expression system for GST fusion protein, will be described. (i) Using cDNA integrated into plasmid pExCell (clone A or FRP) as a template, a cDNA fragment containing an appropriate restriction enzyme site on the 5' side and the 3' side but not containing a signal peptide is obtained by a PCR utilizing the linker primer method. Various heat-resistant DNA polymerases for use in PCR are commercially available; preferably, Pfu DNA polymerase (manufactured by Stratagene) having a high replication rate can be used. The cDNA fragment is separated by agarose gel electrophoresis; the band of interest is cut out; and purification is carried out. For purification, purification kit GENECLEAN II (manufactured by BIO 101, Inc.), which is based on a glass beads method, or the like can be used. (ii) Using a TA cloning kit, e.g., Original TA Cloning Kit (manufactured by Invitrogen BV), the resultant purified cDNA fragment is subcloned into a plasmid (pCRTMII) attached to the kit. (iii) Next, a cDNA fragment is cleaved out with a prescribed restriction enzyme from recombinant plasmid pCRII, and is ligated to pGEX-4T-3 (which is a vector for fusion protein expression) digested with the same restriction enzyme, followed by transformation of an appropriate Escherichia coli (e.g., NM522, TG1, DH5α). (iv) The resultant transformant is cultured and induction with IPTG is carried out, and GST fusion protein is expressed. (v) A lysate of the bacteria is prepared and GST fusion protein is purified by using an anti-GST antibody column. (vi) GST fusion protein is cleaved with thrombin and GST is adsorbed by using an anti-GST antibody column again, and the polypeptide of interest is eluted. Thus, purified clone A polypeptide or FRP can be obtained.

By carrying out western blotting using this purified polypeptide as an antigen, the reactivity with test sera can be examined to indicate disease specificity with respect to RA.

C10 polypeptide and C15 polypeptide can be obtained by chemical synthesis. A peptide chemical synthesis in the state of art mainly employs two methods to provide protection groups for the α-amino group and the side chain functional group, namely, the Boc method, in which the α-amino group is protected by t-butoxycarbonyl (Boc) group and the side chain functional group is protected by a benzyl alcohol type protecting group; and the Fmoc method, in which the α-amino group is protected by a 9-fuluorenylmethoxycarbonyl (Fmoc) group and the side chain functional group is protected by a t-butylalcohol type protecting group. Either synthesis method is applicable, but a solid phase synthesis by the Fmoc method is appropriate because the polypeptide consists of only 10 or 15 amino acids and because of the kind of amino acids included. Specifically, (i) a Fmoc-amino acid corresponding to the C-terminus of a peptide to be synthesized is bound to a support (resin) insoluble to organic solvents, by using an appropriate condensation agent [e.g., PyBOP: Benzotriazol-l-yl-oxy-tris (pyrrolidino) phosphonium hexafluorophosphate is preferable]. Herein, as for amino acids having a side chain functional group, e.g., Thr, Tyr, Glu, Asp, Asn and Ser, such side chain functional groups are preferably protected. (ii) The Fmoc group of the bound amino acid is preferably deprotected by piperidine, which is a secondary amine, and is washed by DMF or the like. (iii) The second Fmoc-amino acid from the C-terminus is bound in the same way as in (i). (iv) The above-mentioned operations (ii) to (iii) are repetitively alternated to sequentially extend the peptide chain from the C-terminus, thereby obtaining a polypeptide-bound resin. This polypeptide-bound resin is dried in vacuo in a desiccator. (v) By stirring the dried polypeptide-bound resin in a weak acid, e.g., 95% TFA (trifluoroacetic acid), deprotection of the polypeptide and release from the resin are effected. (vi) The polypeptide solution in TFA is dropped into diethyl ether or the like to allow the polypeptide to precipitate. After being collected by centrifugation or the like, the polypeptide is dried to yield a crude polypeptide. For synthesis, various auto peptide synthesizers for solid-phase synthesis are commercially sold and available. Moreover, Fmoc-amino acid derivatives for use in the synthesis are all commercially sold and available.

As for purification of synthesized polypeptides, a known method such as chromatography, e.g., ion exchange chromatography or reverse phase liquid chromatography (reverse phase HPLC) can be used alone or in combination. Preferably, reverse phase HPLC can be used. As a column for reverse phase HPLC, various kinds of columns which are commercially available can be used; preferably, 5C18 can be used. The resultant purified polypeptide can be identified as a polypeptide of interest by primary sequencing analysis, amino acid composition analysis, and the like using an amino acid sequencer.

The polypeptide thus-obtained is preferably fresh at the time of use for optimally-retained activity; or when stored at 4°C, it is preferably used within 5 days of storage. Alternatively, a synthesized polypeptide according to the present invention can be cryopreserved by lyophilization. Moreover, a frozen solution of the present polypeptide can be used; preferably, a lyophilized polypeptide is prepared upon use. As described later, C10 polypeptide and C15 polypeptide according to the present invention can be bound to a carrier protein such as BSA, and can be utilized as antigen for ELISA.

In order to allow C10 polypeptide or C15 polypeptide to react with antibodies in sera from RA patients, polypeptide-bound BSA is prepared such that the polypeptide is bound to bovine serum albumin (BSA) by using glutaraldehyde as a crosslinking agent, and a measurement by the ELISA method is enabled by using this as an antigen. Specifically, C10 polypeptide or C15 polypeptide and BSA are dissolved in PBS, and the polypeptide is allowed to be bound to BSA by adding 2% glutaraldehyde while stirring at 4°C. Dialysis to PBS is carried out so as to remove the unreacted polypeptide and reagents. The rest can be carried out according to a usual ELISA method. For example, the resultant polypeptide-bound BSA is dispensed into each well of a microtiter plate (Costar) and left overnight at 4°C. After removing the excessive antigen, blocking is effected with a BSA solution; then, test sera appropriately diluted in a BSA solution are added, and allowed to stand for 2 hours at room temperature. After washing with a washing solution, peroxidase-labeled anti-human IgG antibody is added and allowed to react for an hour at room temperature. After washing with a washing solution, an appropriate peroxidase substrate solution, e.g., an ABTS (manufactured by Zymed) solution adjusted with a 0.1M citric acid buffer (pH 4.2) containing 0.03% hydrogen peroxide is added into each well. After leaving it for 30 minutes at room temperature, the absorbance at 415 nm is measured.

By using this ELISA system, autoantibodies to C10 polypeptide and C15 polypeptide in the sera associated with RA or other autoimmune diseases can be measured, whereby it can be indicated that the antibodies appear specifically in RA patients. In addition, it can be indicated that measuring the antibodies can possibly provide a diagnosis method for RA.

### EXAMPLES

Now, the present invention is described in detail with respect to examples. However, the present invention is not to be limited to these examples.

### [Example 1] Construction of cDNA libraries of synovial cells

### (1) Separation and purification of mRNA

Synovial cells obtained from synovectomy in a knee joint of a RA patient were cut into small pieces and suspended in a DMEM medium (manufactured by Flow) containing 10% fetal calf serum (FCS) and digested for 3 hours with collagenase. The suspended cells were collected and cultured for 2 to 3 weeks in the same medium. During this period, the medium was replaced every 3 to 4 days, and after removing the suspended cells, the adherent cells were used as synovial cells for mRNA preparation. Using TRIZOL 1 reagent (manufactured by Gibco BRL), RNA was prepared from about 10⁸ synovial cells according to the instructions attached to the reagent. The RNA was further placed on a cushion of cesium trifluoroacetate (CsTFA) solution (manufactured by Pharmacia) having a density of 1.51, and ultracentrifugation for 20 hours at 120,000 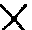 g was performed to collect mRNA in the form of pellets. This mRNA was dissolved into sterilized water and washed by being treated with an equal volume of phenol/chloroform saturated with a buffer. Next, a 1/10 volume of 5M NaCl and 2 volumes of ethanol were added and stirred. By leaving this for 30 minutes at -80°C, mRNA was precipitated and purified. Moreover, Oligotex-dT30 〈super〉 (commercially available from Takara Co., Ltd.) was used for this mRNA according to the attached instructions to purify poly (A)+mRNA.

### (2) Preparation of cDNA libraries

cDNA libraries were prepared by a modified method of Gubler and Hoffman's method [Gene 25 p.263 (1983)]. From the purified poly (A) + mRNA (5µg), single stranded DNA was synthesized with a reverse transcriptase, using (i) random hexamer or (ii) Oligo-dT having a Not I site as a 3' primer. Next, double stranded DNA was synthesized, and using a CHROMA SPIN-400 column (manufactured by Clonetech), cDNA having 200 bases or more was selectively collected. By this operation, low molecular weight nucleotides, enzymes, primers and the like were removed, thereby purifying cDNA. Moreover, after both ends of cDNA were completely blunt ended with T4 DNA polymerase, ligation of EcoR I adapter was performed in the case of (i), or ligation of EcoR I adapter was performed after digestion of Not I in the case of (ii). After the 5' side of cDNA having these restriction enzyme sites was phosphorylated with T4 polynucleotide kinase, adapters, enzymes, and the like were completely removed from both cDNAs by using a CHROMA SPIN-400 column, thereby purifying the cDNAs. The above-mentioned cDNA synthesis step was performed by using TimeSaver cDNA Synthesis Kit (manufactured by Pharmacia). As for the reverse transcriptase, those attached to this kit or SuperScriptTMII RNaseH⁻(manufactured by Giboco BRL) were used. As for ligation, a DNA ligation kit manufactured by Takara Co., Ltd. was used and ligation was carried out according to instructions attached to the kit.

The cDNA was ligated to dephosphorylated λExCell (manufactured by Pharmacia), which is a phage expression vector, at the EcoR I restriction enzyme site in the case of (i), or at EcoR I and Not I sites in the case of (ii). The λExCell having this cDNA integrated therein was packaged into coat protein of phage λ using a Gigapack II packaging kit (manufactured by Stratagene) according to the instructions attached to the kit. The titer of the packaged phage was measured using Escherichia coli NM522 strain (purchased from Stratagene) as a host. As a result, it was presumed to be 50,000 independent clones in the case of (i), and 150,000 independent clones in the case of (ii). With the use of an bromochloroindolyl galactoside(X-gal) indicator, it was shown that 95% of the above were phages having the cDNA inserted therein. The amplification of these recombinant libraries was performed once according to a method attached to the packaging kit, and the phage was collected and stored in 7% dimethyl sulfoxide (DIMSO) at -80°C. These once-amplified libraries were used for antigen screening.

### [Example 2] Preparation of IgG for screening

### (I) Purification of IgG in articular synovial fluid from RA patients

Purification of IgG contained in synovial fluid from RA patients was carried out using Prosep A (manufactured by BoxyBrown), which is a protein A column. After collecting synovial fluid from RA patients, the synovial fluid was diluted by phosphate-buffered saline (PBS) to 3 times and separated by centrifugation for 15 minutes at 5,000×g, and the supernatant was collected to obtain an IgG fraction. After passing this IgG fraction through Prosep A equilibrated by PBS for adsorption of IgG, it was washed with 3 volumes of PBS, and IgG was eluted with 0.1M glycine (pH 3.0). After neutralizing the eluted fraction with 1M Tris-HCl (pH 8.0), it was concentrated to a volume twice that of the original synovial fluid volume. This purified IgG was used as a probe for screening of RA antigens or as a probe for western blotting after obtaining antigens.

### (2) Absorption of anti-Escherichia coli antibodies by Escherichia coli lysate.

Anti-Escherichia coli antibodies were removed from the purified IgG by treating it with a lysate of the host of Escherichia coli NM522 strain, according to the method of Sambrook et al [Molecular Cloning, 12.26 (1989)].

### [Example 3] Cloning from cDNA libraries

### (1) Screening and cloning of cDNA libraries

The cDNA phage libraries prepared in Example 1 were seeded on an NZY agar medium plate (9 cm 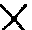 14 cm) to result in 20,000 plaques, and 4 hours of culture at 37°C was carried out, whereby phage plaques appeared. A nitrocellulose membrane (manufactured by Waters) treated with IPTG was placed over the plaques. Concurrently with inducing protein synthesis for 4 hours, the phages were transferred onto the membrane. After washing the membrane onto which the phages had been transferred with PBS three times, blocking was carried out with 5% skim milk (manufactured by DIFCO)/PBS for one hour. After washing with PBS three times, synovial IgG having absorbed the anti-Escherichia coli antibodies obtained in Example 2 was added to allow reaction at 4°C overnight. After washing three times with PBS, this membrane after transfer was treated with an anti-human IgG antibody labeled with horseradish peroxidase (HRP) for one hour, and washed with PBS three times. Thereafter, positive phages binding to IgG were detected by using an ECR kit (manufactured by Amersham), according to the instructions attached to the kit. After collection of the positive phages, similar screening was carried out 3 times to finally obtain 2 kinds of 100% positive phage clones.

The cloned phages were added to Escherichia coli NM522 strain, which had previously been cultured for 20 minutes at 39°C in an NZCYM medium containing 50 µg/ml of spectinomycin, and another 20 minutes of incubation at 39°C was performed to convert the phage DNA into circular phagemids (pExCell). The conversion was stopped by adding 1M sodium citrate, and a 2YT medium containing spectinomycin was added and cultured with gentle shaking at 37°C for 1.5 hours. The NM522 strain containing pExCell was further seeded on an LB plate containing ampicillin for a culture, whereby Escherichia coli containing the gene of interest was cloned. This step of conversion into pExCell was carried out according to the description attached to λExCell cloning vector (manufactured by Pharmacia). After collecting this pExCell plasmid from the NM522 strain, this plasmid was used to transform a DH5α strain (purchased from TOYOBO Co., Ltd.), whereby a stable plasmid expressing strain was obtained.

### (2) Determination of the nucleotide sequence

The cDNA sequence in the two kinds of plasmids cloned in (1) of Example 3 were subjected to reaction using a Taq Dyedeoxy Terminator Cycle Sequencing Kit manufactured by Applied Biosystems, and the nucleotide sequence was determined by fluorescent detection using an Applied Biosystems 373A DNA sequencer. The putative amino acid sequences of the cloned autoantigens are shown in SEQ ID NO: 1 and SEQ ID NO: 3 of the Sequence Listing, whereas the nucleotide sequences of their respective open reading frames are shown in SEQ ID NO: 2 and SEQ ID NO: 4.

### (3) Homology analysis of partial nucleotide sequences

For the two kinds of cDNA nucleotide sequences obtained from (2) in Example 3 above, a homology search with respect to all nucleotide sequences included in a known database DDBJ was carried out by using a FASTA program of Lipman and Pearson [Proc. Natl. Aca. Sci. USA 85, p.2444 (1988)]. As a result, the cDNA nucleotide sequence shown in SEQ ID NO: 2 of the Sequence Listing did not match to any nucleotide sequence, and thus it was indicated to be a novel sequence. Gp130, which is a β chain of Interleukin 6 receptor, was found as a polypeptide having homology with the amino acid sequence (SEQ ID NO: 1 of the Sequence Listing) deduced from this nucleotide sequence. Compared with the gp130 sequence, it was found that 83 bases from 1229 to 1311 of the reported gp130 nucleotide sequence [Hibi et al., Cell 63, p.1149 (1990)] were deleted by splicing in the nucleotide sequence encoding the polypeptide according to the present invention; a stop codon appeared through frame shifting; and consequently, the amino acid sequence changed from Arg (325)-Pro-Ser-Lys-Ala-Pro(330)-Ser-Phe-Trp-Tyr--, which would continue to position 918, to an entirely different amino acid sequence of Asn (325)-Ile-Ala-Ser-PheOH (329), resulting in a short polypeptide up to amino acid 329. The nucleotide sequence shown in SEQ ID NO: 4 of the Sequence Listing matched the nucleotide sequence from positions 16 to 1150 of the nucleotide sequence of FRP [Zwijsen et al., Eur. J. Biochem. 225, p.937 (1994)] and contained the entire amino acid sequence (SEQ ID NO: 3 of the Sequence Listing). Both are completely novel as rheumatism antigens.

### [Example 4] Reactivity between IgG from RA patients and autoantigens

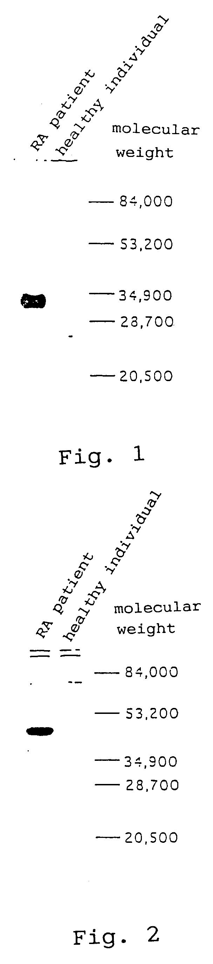

The two kinds of Escherichia coli NM522 strain including the antigenic plasmid obtained in Example 3 above were each cultured in 5 ml of a LB medium to which ampicillin had been added, and IPTG was added to 1mM when O.D. 600 was at 0.5, and another 3 hours of culture was carried out to induce protein synthesis. Escherichia coli was collected and made soluble with SDS. After SDS-polyacrylamide electrophoresis, western blotting was carried out. The western blotting was carried out by using as a probe the synovial IgG from RA patients obtained from (1) of Example 2 above, and using HRP-labeled anti-human IgG as a secondary antibody, with the use of an ECR kit (manufactured by Amersham) according to a method attached to the kit. Exemplary results of this are shown in Fig.1 and Fig.2 respectively. Positive patients were detected when using clone A polypeptide according to the present invention (Fig.1), and the existence of positive patients were confirmed when using FRP (Fig.2).

### [Example 5] Preparation and expression of an expression vector for Escherichia coli.

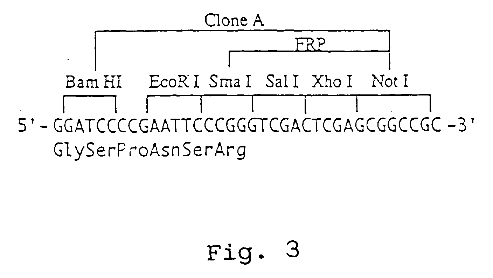

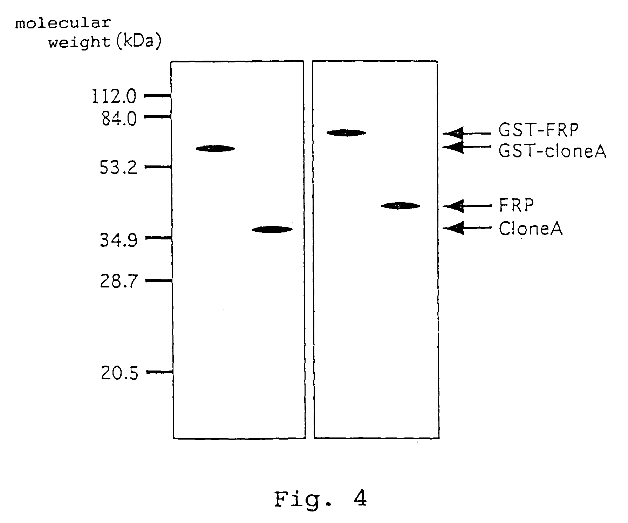

The preparation, production of a GST fusion protein expression vector for Escherichia coli and the purification of the polypeptide of interest were carried out by using a Glutathione S-transferase (GST) Gene Fusion System manufactured by Pharmacia, according to the instructions attached to the kit.
(1) In order to express cDNA products in Escherichia coli in large quantities and in a form that allows easier purification, cDNA was integrated to plasmid pGEX-4T-3 (manufactured by Pharmacia) so that the translation product of the cDNA would be a fusion protein with glutathione S-transferase (GST). At this time, first, the cDNA was subjected to a PCR utilizing the linker primer method, with the cDNA being integrated in the plasmid pExCell, to give a cDNA fragment containing the below-described restriction enzyme site both on the 5' side and the 3' side and containing no signal peptides. After agarose gel electrophoresis, the resultant PCR product was purified from the cut-out gel by GENECLEAN II (manufactured by BIO 101, Inc.) according to a method attached to the kit. The purified cDNA was ligated to plasmid pCRII by using an Original TA Cloning Kit (manufactured by Invitrogen BV) according to a method attached to the kit. Next, Escherichia coli TOP10F' was transformed and the plasmid was collected. Then, the collected plasmid was cleaved by a predetermined restriction enzyme and separated by agarose gel electrophoresis, whereafter the gel containing the cDNA of interest was cut out and purified by GENECLEAN II. On the other hand, pGEX-4T-3 was cleaved by the same restriction enzyme and separated by electrophoresis, whereafter the vector arm side was collected and purified in the same manner. The insert cDNA was ligated to the vector arm, and after transformation of Escherichia coli NM522 strain, the plasmid was collected and the expression vector of interest was obtained.
   pGEX-4T-3 cloning sites of clone A and FRP are shown in Fig. 3. As for clone A, among pGEX-4T-3 multicloning sites, BamHI and Not I were selected on the 5' side as viewed from the inserted fragment so as to conserve the antigenicity of the product of cDNA as much as possible. Specifically, after thrombin digestion, two amino acids, i.e., glycine and serine, were added in this order from the N terminus of the product of cDNA cut out from GST, without substitution of any amino acids (SEQ ID NO: 5). As for FRP, on the other hand, SmaI on the 5' side and Not I on the 3' side were selected, so as to similarly conserve the antigenicity of the product of cDNA as much as possible. Specifically, after thrombin digestion, 6 amino acids, i.e., glycine, serine, proline, asparagine, serine, arginine were added in this order from the N-terminus of the product of cDNA cut out from GST, without substitution of any amino acids (SEQ ID NO: 6).
(2) Hereinafter, a primer and a method used for the PCR will be described.
   Used as a clone A primer were 5'-AAGGATCCGAACTTCTAGATCCATGTGG-3' (SEQ ID NO: 7) and 5'-TTGCGGCCGCTCAAAAGGAGGCAATGTTAT-3' (SEQ ID NO: 8). As a FRP primer, 5'-AACCCGGGAGGAAGAGCTAAGGAGCAA-3' (SEQ ID NO: 9) and 5'-TTGCGGCCGCTGTGCCTCCTCATTAGATCT-3' (SEQ ID NO: 10) were used.
   As a heat resistant DNA polymerase, Pfu DNA polymerase (Cloned Pfu DNA polymerase, manufactured by Stratagene) having a high replication rate was used. The composition of the reaction solution was as follows. Fifty nanograms of a plasmid having cDNA as a template, primers of 15 pmol each, 5 µl of a 10 times concentration solution attached to Pfu DNA polymerase as a buffer, and 4 µl of 2.5 mM dNTPs Mixture (manufactured by Takara Co., Ltd.) as dNTPs were added, resulting in 49 µl with sterilized distilled water; finally to this, 1 µl of Pfu DNA polymerase was added. After mineral oil (manufactured by Aldrich Chem. Company Inc.) was placed on the above-mentioned reaction solution, the reaction solution was set in a DNA Thermal Cycler PJ2000 (manufactured by Perkin Elmer) to perform a PCR under the conditions where 3 minutes at 94°C was followed by 25 cycles each including 30 seconds at 94°C, 30 seconds at 55°C and 2 minutes at 72°C. Because Pfu DNA polymerase was used, Taq DNA polymerase (manufactured by Nippon Gene) was added after the PCR so as not to undermine the TA cloning efficiency, according to the description in the instructions attached to the Original TA Cloning Kit, and a reaction was effected at 72°C for 10 minutes. Immediately after this, a step was additionally included to inactivate DNA polymerase by a phenol-chloroform treatment.
(3) Expression and purification of recombinant clone A polypeptide and FRP
   The expression and purification of recombinant clone A polypeptide and FRP were carried out according to a manual attached to the kit in above-mentioned manner. The method is described briefly.
   Escherichia coli transformed with pGEX in which cDNA encoding each polypeptide was integrated was cultured at 37°C in a 2x YT medium containing 100 µg/ml of ampicillin until OD600 reached 0.6 to 0.8. IPTG was added to a final concentration of 1mM, and a fusion protein was expressed by another 1 to 2 hours of culture. The bacterial bodies were collected by centrifugation, and the samples were disrupted by a sonicator in cold PBS. 20% Triton X-100 was added to attain a final concentration of 1%, and 30 minutes of stirring as carried out, thereby dissolving GST fusion protein. After centrifugation at 12,000×g at 4°C for 10 minutes and collecting the supernatant, filtration with a 0.45 µm filter was carried out. After the filtrate was passed through a Glutathione Sepharose 4B RedPack column for adsorption of GST fusion protein, the fusion protein was eluted by using a glutathione elution solution. A thrombin solution was added to this eluate and incubated for 15 hours at 22°C to 25°C to separate the polypeptides of interest from the GST polypeptide. Next, glutathione was first removed by dialyzing this solution. Subsequently, the solution was passed through a Glutathione Sepharose 4B RedPack column for adsorption of GST, thereby eluting and collecting the polypeptides of interest. These purified polypeptides showed a single band by SDS-PAGE, and were shown by western blotting to react with synovial IgG from RA patients (Fig.4).

### [Example 6] Measurement of anti-FRP antibodies in patients of rheumatism related diseases

By western blotting using the recombinant FRP obtained in Example 5, the anti-FRP antibodies in the sera from patients of rheumatism related diseases were measured. SDS-PAGE of 0.5 µg/lane of purified antigens was carried out with 12.5% polyacrylamide gel, followed by transfer onto an Immobilon membrane (manufactured by Millipore). After blocking this Immobilon membrane with 5% skim milk/PBS, it was allowed to react with sera from autoimmune disease patients or sera from healthy individuals (diluted to 400× with 5% skim milk/PBS) overnight at 4°C. After washing with PBS-Tween 20, goat anti-human IgG antibody (manufactured by Cappel) labeled with 2000× diluted peroxidase as a secondary antibody was allowed to react for 1 hour. After washing with PBS-Tween 20, detection was carried out in an ECL system. The sera from rheumatism related patients used included: 67 cases of RA patients (RA), 51 cases of systemic lupus erythematosus, 18 cases of scleroderma, 10 cases of Sjö gren's syndrome, 13 cases of polymyositis/dermatomyositis and 30 cases of healthy individuals. The results are shown in Table 1.

**Table 1**

| Percentage of positives of anti-FRP antibodies with sera from patients of rheumatism related diseases | | | |
|---|---|---|---|
| Disease | n | Number of positives | Percentage of positives |
| Rheumatoid Arthritis (RA) | 67 | 20 | 29.9 |
| Systemic Lupus Erythematosus (SLE) | 51 | 5 | 9.8 |
| Scleroderma (SSc) | 18 | 3 | 17 |
| Sjogren's syndrome (SjS) | 10 | 1 | 10 |
| polymyositis/dermatomyositis(PM/DM) | 13 | 0 | 0 |
| healthy individuals | 30 | 0 | 0 |

From these results, it is indicated that anti-FRP antibodies emerge in an RA-specific manner and therefore can be used for diagnosis of RA.

Furthermore, the anti-FRP antibodies in RA articular synovial fluid (18 cases) and osteoarthritis (OA) synovial fluid (15 cases) were also measured in the same manner. As a result, the percentage of positives for RA showed an efficient detection of 44.4%, as shown in Table 2, while no instances were detected for OA. From these results, too, it was shown that anti-FRP antibody measurements can be used for diagnosis of RA.

**Table 2**

| Percentage of positives of anti-FRP antibodies in articular fluid of rheumatism disease and OA disease | | | |
|---|---|---|---|
| Disease | n | Number of positives | Percentage of positives |
| Rheumatoid Arthritis (RA) | 18 | 8 | 44.4 |
| Osteoarthritis (OA) | 15 | 0 | 0 |

### [Example 7] Peptide synthesis by solid-phase method

Synthesis of C10 polypeptide and C15 polypeptide was carried out by the Fmoc method by using a peptide synthesizer (Shimadzu Seisakusyo Ltd., PSSM-8 type). A peptide chain was sequentially extended from TGS-AC-Fmoc-Phe, which is a support to which Phe at the C terminus is bound (0.22 mmol/g resin, total amount 100 mg, manufactured by Shimadzu corp.), towards the N terminus direction, by repeating the reaction for removing the Fmoc group and condensation reaction. Specifically, the Fmoc group, which is a protecting group of the α-amino group, was removed with 30% piperidine/DMF twice for two minutes each, and washing with N'N-dimethylformamide (DMF) was carried out 5 times for five minutes each. A condensation reaction was carried out for 30 minutes by PyBOP [benzotriazol-1-yl-oxy-tris (pyrrolidino)phosphonium hexafluorophosphate] (manufactured by Watanabe Chemistry) in the presence of 1-hydroxybenzotriazole (HOBt, manufactured by Watanabe Chemistry), then washing by DMF was repeated (5 minutes, 5 times). By repeating the reaction for removing the Fmoc group and the subsequent condensation reactions of amino acids, the polypeptide of interest was synthesized. These syntheses were carried out by automatic operation with a synthesis program for the Fmoc method, which was loaded in the above-mentioned peptide synthesizer. As for Fmoc-amino acids, Fmoc-L-Ala, Fmoc-L-Asn (Trt), Fmoc-L-Asp (OtBu), Fmoc-L-Glu (OtBu), Fmoc-L-Ile, Fmoc-L-Thr (tBu), Fmoc-L-Ser (tBu), Fmoc-L-Tyr (tBu) and Fmoc-Gly were used. For each of these, about 10× molar amount based on the substrate was used. (Herein, Trt, OtBu, Boc, and tBu represent a trityl group, tert-butyl ester, a butyloxycarbonyl group and a tert-butyl group, respectively).

After synthesis was completed, a washing was carried out with 10 ml of diethyl ether per 22 µmol scale of peptide binding resin, and then 2 ml of 95% TFA was added, and deprotection and separation from the resin were carried out while stirring at room temperature for 2 hours. Next, after dropping this solution slowly into a centrifuge tube containing 40 ml of cold diethyl ether while stirring, the polypeptide was precipitated by allowing it to stand in icy water for 30 minutes. After centrifugation (5,000 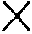 g 10 minutes, 4°C), the supernatant was discarded, and 45 ml of cold diethyl ether was added to the resultant precipitate, and stirred well. After allowing it to stand for 5 minutes in icy water, the precipitate was collected by another centrifugation. After repeating this operation 3 times, the precipitate was dried in vacuo, whereby the polypeptide of interest was obtained.

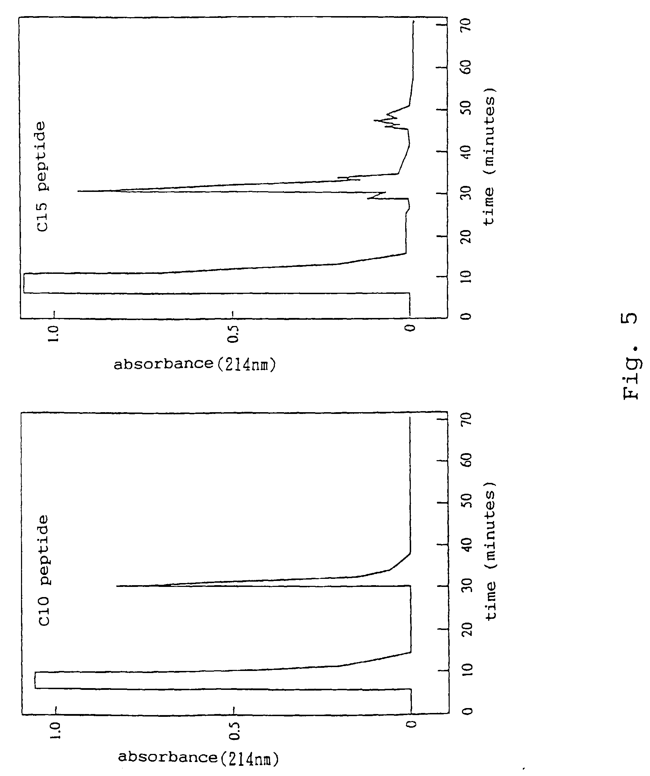

The purification of the resultant crude polypeptide was carried out by reverse phase HPLC as follows. The precipitate was dissolved in 5 ml of a 0.1% aqueous solution of trifluoroacetic acid. Filtration through a 0.45 µm filter was carried out, and the resultant filtrate was subjected to HPLC. For HPLC, a Model LC-8A system (manufactured by Shimadzu Seisakusyo Ltd.) was used. For the column, a Cosmosil 5C18 (20 x 250 nm) (manufactured by nacalai tesque) of a reverse phase type was used. As a mobile phase, 0.1% TFA and 50% (v/v) acetonitrile/0.1% TFA were used as an A solution and a B solution, respectively. Elution was carried out based on a linear concentration gradient from a 0% B solution to a 100% B solution. The elution patterns are shown in Fig. 5-1 (C10 polypeptide) and Fig. 5-2 (C15 polypeptide). The polypeptide eluted fraction was collected and lyophilized to give a purified polypeptide. The resultant polypeptide was analyzed by a gas-phase protein sequencer 477 type (manufactured by Applied Biosystems), whereby it was confirmed that the polypeptide having the amino acid sequence of interest had been obtained.

### [Example 8] Construction of an ELISA system using C10 polypeptide or C15 polypeptide.

### (1) Binding C10 polypeptide or C15 polypeptide to bovine serum albumin

By using glutaraldehyde, C10 polypeptide or C15 polypeptide was bound to BSA according to the following method: After 5 mg of BSA was dissolved into 5 ml of PBS, 2 mg of each polypeptide was added. Five milliliters of a 2% aqueous solution of glutaraldehyde was slowly dropped into this solution at 4°C while being stirred, and the reaction was effected for 1 hour with stirring. Next, 100 mg of tetrahydro sodium borate was added and the solution was allowed to stand for 1 hour. The resultant reacted solution was dialyzed to PBS, whereby BSA-bound polypeptide was obtained.

### (2) ELISA using BSA-bound C10 polypeptide or C15 polypeptide

The antibody titers of autoantibodies against clone A polypeptide in sera from patients were determined by the following ELISA: 50 µl of a solution of BSA-bound polypeptide, prepared to 2 µg/ml in PBS, was placed into each well of a- microtiter plate (Costar), and left overnight at 4°C. After washing twice with PBS, 100 µl of PBS containing 5% BSA was added and left at room temperature for 1 hour. After washing twice with PBS containing 0.05% Tween-20, 50 µl of sera diluted to 100 times with PBS containing 5% BSA was added, and allowed to react at room temperature for 2 hours. After washing 3 times with PBS containing 0.05% Tween-20, 50µl of goat anti-human IgG antibody (Cappel) labeled with peroxidase diluted to 2000 times with PBS containing 5% BSA was added, and allowed to react at room temperature for 1 hour. After washing 5 times with PBS containing 0.05% Tween-20, 50µl of an ABTS solution (manufactured by Zymed) prepared by using a 0.1M citrate buffer (pH 4.2) containing 0.03% hydrogen peroxide was added into each well. After being left at room temperature for 30 minutes, the absorbance (OD) of each well at 415 nm was measured with a microplate reader (manufactured by Corona Electron, MTP32).

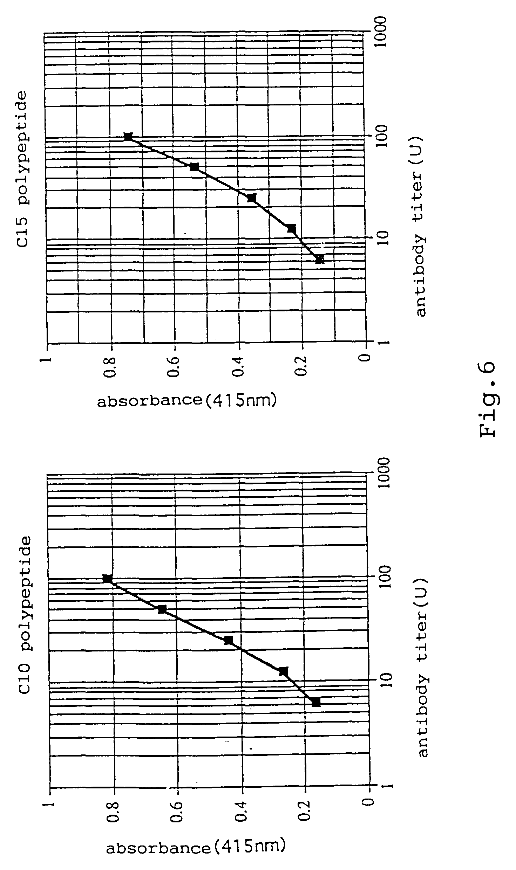

The antibody titer of the autoantibody against clone A in each serum was calculated, based on a standard curve showing the relationship between the antibody titers and the OD values obtained from the sera of an RA patient showing a high antibody titer, where the sera of the RA patient was defined as 100 units (U). The standard curves for C10 polypeptide and C15 polypeptide are shown in Fig.6-1 and Fig.6-2, respectively. These curves made possible the measurement of the polypeptide antibody in an ELISA employing the polypeptide as an antigen.

### [Example 9] Measurement of anti-C10 polypeptide antibodies in rheumatism related disease patients

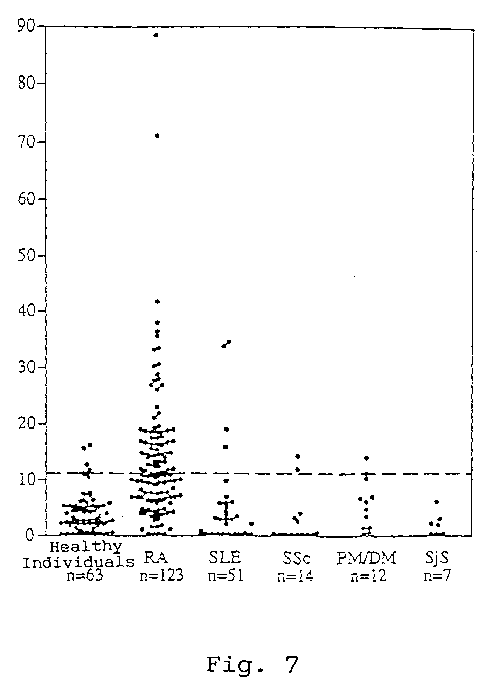

The measurement of anti-C10 polypeptide antibodies in rheumatism related diseases was carried out by an ELISA system using C10 polypeptide as an antigen. The disease used are: 123 cases of RA (RA), 51 cases of systemic lupus erythematosus, 14 cases of scleroderma, 7 cases of Sjögren's syndrome, 12 cases of polymyositis/dermatomyositis, and 63 cases of healthy individuals. A scatter diagram for each disease of the measured results is shown in Fig.7. The percentages of positives are shown in Table 3.

**Table 3**

| Percentage of positives of anti-C10 peptide antibodies with sera from patients of rheumatism related diseases | | | |
|---|---|---|---|
| Disease | n | Number of positives | Percentage of positives |
| Rheumatoid Arthritis (RA) | 123 | 60 | 48.8 |
| Systemic Lupus Erythematosus (SLE) | 51 | 4 | 7.8 |
| Scleroderma (SSc) | 14 | 2 | 14.3 |
| Sjögren's syndrome (SjS) | 7 | 0 | 0 |
| polymyositis/dermatomyositis(PM/DM) | 12 | 1 | 8.3 |
| healthy individuals | 63 | 4 | 6.3 |

Any instance was defined as positive that showed a value which showed a higher value than the average of antibody titer of a healthy individual + (2 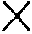 standard deviation). From these results, it was shown that the measured values of RA patients are statistically significant compared with patients of other diseases and healthy individuals and also the measurements of the polypeptide antibody are RA specific. Accordingly, it was shown that the measurements of the polypeptide antibody can be used for RA diagnosis.

### INDUSTRIAL APPLICABILITY

The Two kinds of antigens obtained according to the present invention are novel as rheumatism antigens, and are quite significant in the development of diagnostics. Moreover, these antigens are expressed in articular synovial cells from RA patients, indicative of a possibility that studying the significance of these antigens being involved in RA pathoses can lead to the development of therapeutics.

SEQUENCE LISTING

SEQ ID NO: 1
SEQUENCE LENGTH: 329 amino acids
SEQUENCE TYPE: amino acid
TOPOLOGY: linear
MOLECULE TYPE: protein
SEQUENCE DESCRIPTION: SEQ ID NO: 1:


1               5                   10                  15

            20                  25                  30

        35                  40                  45

    50                  55                  60

65                  70                  75                  80

                85                  90                  95

            100                 105                 110

        115                 120                 125

    130                 135                 140


145                 150                 155                 160

                165                 170                 175

            180                 185                 190

        195                 200                 205

    210                 215                 220

225                 230                 235                 240

                245                 250                 255

            260                 265                 270

        275                 280                 285

    290                 295                 300

305                 310                 315                 320

                325

SEQ ID NO: 2
SEQUENCE LENGTH: 990 base pairs
SEQUENCE TYPE: nucleic acid
STRANDEDNESS: single
TOPOLOGY: linear
MOLECULE TYPE: cDNA to mRNA
SEQUENCE DESCRIPTION: SEQ ID NO: 2:

ATGTTGACGT TGCAGACTTG GGTAGTGCAA GCCTTGTTTA TTTTCCTCAC CACTGAATCT 60
ACAGGTGAAC TTCTAGATCC ATGTGGTTAT ATCAGTCCTG AATCTCCAGT TGTACAACTT 120
CATTCTAATT TCACTGCAGT TTGTGTGCTA AAGGAAAAAT GTATGGATTA TTTTCATGTA 180
AATGCTAATT ACATTGTCTG GAAAACAAAC CATTTTACTA TTCCTAAGGA GCAATATACT 240
ATCATAAACA GAACAGCATC CAGTGTCACC TTTACAGATA TAGCTTCATT AAATATTCAG 300
CTCACTTGCA ACATTCTTAC ATTCGGACAG CTTGAACAGA ATGTTTATGG AATCACAATA 360
ATTTCAGGCT TGCCTCCAGA AAAACCTAAA AATTTGAGTT GCATTGTGAA CGAGGGGAAG 420
AAAATGAGGT GTGAGTGGGA TGGTGGAAGG GAAACACACT TGGAGACAAA CTTCACTTTA 480
AAATCTGAAT GGGCAACACA CAAGTTTGCT GATTGCAAAG CAAAACGTGA CACCCCCACC 540
TCATGCACTG TTGATTATTC TACTGTGTAT TTTGTCAACA TTGAAGTCTG GGTAGAAGCA 600
GAGAATGCCC TTGGGAAGGT TACATCAGAT CATATCAATT TTGATCCTGT ATATAAAGTG 660
AAGCCCAATC CGCCACATAA TTTATCAGTG ATCAACTCAG AGGAACTGTC TAGTATCTTA 720
AAATTGACAT GGACCAACCC AAGTATTAAG AGTGTTATAA TACTAAAATA TAACATTCAA 780
TATAGGACCA AAGATGCCTC AACTTGGAGC CAGATTCCTC CTGAAGACAC AGCATCCACC 840
CGATCTTCAT TCACTGTCCA AGACCTTAAA CCTTTTACAG AATATGTGTT TAGGATTCGC 900
TGTATGAAGG AAGATGGTAA GGGATACTGG AGTGACTGGA GTGAAGAAGC AAGTGGGATC 960
ACCTATGAAG ATAACATTGC CTCCTTTTGA 990

SEQ ID NO: 3
SEQUENCE LENGTH: 308 amino acids
SEQUENCE TYPE: amino acid
TOPOLOGY: linear
MOLECULE TYPE: protein
SEQUENCE DESCRIPTION: SEQ ID NO: 3:
Met Trp Lys Arg Trp Leu Ala Leu Ala Leu Ala Leu Val Ala Val Ala
1               5                   10                  15
Trp Val Arg Ala Glu Glu Glu Leu Arg Ser Lys Ser Lys Ile Cys Ala
            20                  25                  30
Asn Val Phe Cys Gly Ala Gly Arg Glu Cys Ala Val Thr Glu Lys Gly
        35                  40                  45
Glu Pro Thr Cys Leu Cys Ile Glu Gln Cys Lys Pro His Lys Arg Pro
    50                  55                  60
Val Cys Gly Ser Asn Gly Lys Thr Tyr Leu Asn His Cys Glu Leu His
65                  70                  75                  80
Arg Asp Ala Cys Leu Thr Gly Ser Lys Ile Gln Val Asp Tyr Asp Gly
                85                  90                  95
His Cys Lys Glu Lys Lys Ser Val Ser Pro Ser Ala Ser Pro Val Val
            100                 105                 110
Cys Tyr Gln Ser Asn Arg Asp Glu Leu Arg Arg Arg Ile Ile Gln Trp
        115                 120                 125
Leu Glu Ala Glu Ile Ile Pro Asp Gly Trp Phe Ser Lys Gly Ser Asn
    130                 135                 140
Tyr Ser Glu Ile Leu Asp Lys Tyr Phe Lys Asn Phe Asp Asn Gly Asp
145                 150                 155                 160
Ser Arg Leu Asp Ser Ser Glu Phe Leu Lys Phe Val Glu Gln Asn Glu
                165                 170                 175

Thr Ala Ile Asn Ile Thr Thr Tyr Pro Asp Gln Glu Asn Asn Lys Leu
            180                 185                 190
Leu Arg Gly Leu Cys Val Asp Ala Leu Ile Glu Leu Ser Asp Glu Asn
        195                 200                 205
Ala Asp Trp Lys Leu Ser Phe Gln Glu Phe Leu Lys Cys Leu Asn Pro
    210                 215                 220
Ser Phe Asn Pro Pro Glu Lys Lys Cys Ala Leu Glu Asp Glu Thr Tyr
225                 230                 235                 240
Ala Asp Gly Ala Glu Thr Glu Val Asp Cys Asn Arg Cys Val Cys Ala
                245                 250                 255
Cys Gly Asn Trp Val Cys Thr Ala Met Thr Cys Asp Gly Lys Asn Gln
            260                 265                 270
Lys Gly Ala Gln Thr Gln Thr Glu Glu Glu Met Thr Arg Tyr Val Gln
        275                 280                 285
Glu Leu Gln Lys His Gln Glu Thr Ala Glu Lys Thr Lys Arg Val Ser
    290                 295                 300
Thr Lys Glu Ile
305

SEQ ID NO: 4
SEQUENCE LENGTH: 926 base pairs
SEQUENCE TYPE: nucleic acid
STRANDEDNESS: single
TOPOLOGY: linear
MOLECULE TYPE: cDNA to mRNA
SEQUENCE DESCRIPTION: SEQ ID NO: 4:

ATGTGGAAAC GCTGGCTCGC GCTCGCGCTC GCGCTGGTGG CGGTCGCCTG GGTCCGCGCC 60
GAGGAAGAGC TAAGGAGCAA ATCCAAGATC TGTGCCAATG TGTTTTGTGG AGCCGGCCGG 120
GAATGTGCAG TCACAGAGAA AGGGGAACCC ACCTGTCTCT GCATTGAGCA ATGCAAACCT 180
CACAAGAGGC CTGTGTGTGG CAGTAATGGC AAGACCTACC TCAACCACTG TGAACTGCAT 240
CGAGATGCCT GCCTCACTGG ATCCAAAATC CAGGTTGATT ACGATGGACA CTGCAAAGAG 300
AAGAAATCCG TAAGTCCATC TGCCAGCCCA GTTGTTTGCT ATCAGTCCAA CCGTGATGAG 360
CTCCGACGTC GCATCATCCA GTGGCTGGAA GCTGAGATCA TTCCAGATGG CTGGTTCTCT 420
AAAGGCAGCA ACTACAGTGA AATCCTAGAC AAGTATTTTA AGAACTTTGA TAATGGTGAT 480
TCTCGCCTGG ACTCCAGTGA ATTCCTGAAG TTTGTGGAAC AGATGAAACT GCCATCAATA 540
TTACAACGTA TCCAGACCAG GAGAACAACA AGTTGCTTAG GGGACTCTGT GTTGATGCTC 600
TCATTGAACT GTCTGATGAA AATGCTGATT GGAAACTCAG CTTCCAAGAG TTTCTCAAGT 660
GCCTCAACCC ATCTTTCAAC CCTCCTGAGA AGAAGTGTGC CCTGGAGGAT GAAACGTATG 720
CAGATGGAGC TGAGACCGAG GTGGACTGTA ACCGCTGTGT CTGTGCCTGT GGAAATTGGG 780
TCTGTACAGC CATGACCTGT GACGGAAAGA ATCAGAAGGG GGCCCAGACC CAGACAGAGG 840
AGGAGATGAC CAGATATGTC CAGGAGCTCC AAAAGCATCA GGAAACAGCT GAAAAGACCA 900
AGAGAGTGAG CACCAAAGAG ATCTAA 926

SEQ ID NO: 5
SEQUENCE LENGTH: 10 amino acids
SEQUENCE TYPE: amino acid
TOPOLOGY: linear
MOLECULE TYPE: polypeptide
SEQUENCE DESCRIPTION: SEQ ID NO: 5:
Ile Thr Tyr Glu Asp Asn Ile Ala Ser Phe
1               5                   10

SEQ ID NO: 6
SEQUENCE LENGTH: 15 amino acids
SEQUENCE TYPE: amino acid
TOPOLOGY: linear
MOLECULE TYPE: polypeptide
SEQUENCE DESCRIPTION: SEQ ID NO: 6:
Glu Glu Ala Ser Gly Ile Thr Tyr Glu Asp Asn Ile Ala Ser Phe
1               5                   10                  15

SEQ ID NO: 7
SEQUENCE LENGTH: 28 base pairs
SEQUENCE TYPE: nucleic acid
STRANDEDNESS: single
MOLECULE TYPE: synthetic DNA
SEQUENCE DESCRIPTION: SEQ ID NO: 7:
AAGGA TCCGA ACTTC TAGAT CCATG TGG 28

SEQ ID NO: 8
SEQUENCE LENGTH: 30 base pairs
SEQUENCE TYPE: nucleic acid
STRANDEDNESS: single
MOLECULE TYPE: synthetic DNA
SEQUENCE DESCRIPTION: SEQ ID NO: 8:
TTGCG GCCGC TCAAA AGGAG GCAAT GTTAT 30

SEQ ID NO: 9
SEQUENCE LENGTH: 27 base pairs
SEQUENCE TYPE: nucleic acid
STRANDEDNESS: single
MOLECULE TYPE: synthetic DNA
SEQUENCE DESCRIPTION: SEQ ID NO: 9:
AACCC GGGAG GAAGA GCTAA GGAGC AA 27

SEQ ID NO: 10
SEQUENCE LENGTH: 30 base pairs
SEQUENCE TYPE: nucleic acid
STRANDEDNESS: single
MOLECULE TYPE: synthetic DNA
SEQUENCE DESCRIPTION: SEQ ID NO: 10:
TTGCG GCCGC TGTGC CTCCT CATTA GATCT 30

## Claims

1. A polypeptide shown in SEQ ID NO: 5 of the Sequence Listing, a polypeptide including the amino acid sequence of SEQ ID NO: 5, or a polypeptide including a substitution, deletion or addition in at least one amino acid in the amino acid sequence of SEQ ID NO: 5, the polypeptide binding to an antibody which is specific to rheumatoid arthritis patients.

2. A polypeptide shown in SEQ ID NO: 6 of the Sequence Listing, a polypeptide including the amino acid sequence of SEQ ID NO: 6, or a polypeptide including a substitution, deletion or addition in at least one amino acid in the amino acid sequence of SEQ ID NO: 6, the polypeptide binding to an antibody which is specific to rheumatoid arthritis patients.

3. A polypeptide including a portion of the amino acid sequence shown in SEQ ID NO: 1 of the Sequence Listing, the polypeptide binding to an antibody which is specific to rheumatoid arthritis patients.

4. A polypeptide including the amino acid sequence shown in SEQ ID NO: 1 of the Sequence Listing, the polypeptide binding to an antibody which is specific to rheumatoid arthritis patients.

5. A polypeptide having the amino acid sequence shown in SEQ ID NO: 1 of the Sequence Listing, or a polypeptide including a substitution, deletion or addition in at least one amino acid in the amino acid sequence, the polypeptide binding to an antibody which is specific to rheumatoid arthritis patients.

6. A polypeptide including a substitution, deletion or addition in at least one amino acid in the amino acid sequence shown in SEQ ID NO: 3 of the Sequence Listing, the polypeptide binding to an antibody which is specific to rheumatoid arthritis patients.

7. A DNA encoding a polypeptide binding to an antibody which is specific to rheumatoid arthritis patients.

8. A DNA according to claim 7, wherein the DNA encodes any one of the following polypeptides:
(a) a polypeptide shown in SEQ ID NO: 5 of Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to rheumatoid arthritis patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to rheumatoid arthritis patients;
(g) a polypeptide having the amino acid sequence shown in SEQ ID NO: 1 of the Sequence Listing, the polypeptide binding to an antibody which is specific to rheumatoid arthritis patients;
(h) a polypeptide which is a fragment of the amino acid sequence shown in SEQ ID NO: 1 of the Sequence Listing, the polypeptide binding to an antibody which is specific to rheumatoid arthritis patients;
(i) a polypeptide including the amino acid sequence shown in SEQ ID NO: 1 of the Sequence Listing, the polypeptide binding to an antibody which is specific to rheumatoid arthritis patients;
(j) a polypeptide including a substitution, deletion or addition in at least one amino acid in the amino acid sequence shown in SEQ ID NO: 1 of the Sequence Listing, the polypeptide binding to an antibody which is specific to rheumatoid arthritis patients;
(k) a polypeptide which is a fragment of the amino acid sequence shown in SEQ ID NO: 3 of the Sequence Listing, the polypeptide binding to an antibody which is specific to rheumatoid arthritis patients;
(l) a polypeptide including the amino acid sequence shown in SEQ ID NO: 3 of the Sequence Listing, the polypeptide binding to an antibody which is specific to rheumatoid arthritis patients; and
(m) a polypeptide including a substitution, deletion or addition in at least one amino acid in the amino acid sequence shown in SEQ ID NO: 3 of the Sequence Listing, the polypeptide binding to an antibody which is specific to rheumatoid arthritis patients.

9. A DNA according to claim 7, wherein the DNA is a sequence shown in SEQ ID NO: 2 or SEQ ID NO: 4 of the Sequence Listing.

10. An expression vector including a DNA encoding a polypeptide binding to an antibody which is specific to rheumatoid arthritis patients.

11. An expression vector according to claim 10, wherein the DNA is a DNA encoding any one of the following polypeptides:
(a) a polypeptide shown in SEQ ID NO: 5 of Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to rheumatoid arthritis patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to rheumatoid arthritis patients;
(g) a polypeptide having the amino acid sequence shown in SEQ ID NO: 1 of the Sequence Listing, the polypeptide binding to an antibody which is specific to rheumatoid arthritis patients;
(h) a polypeptide which is a fragment of the amino acid sequence shown in SEQ ID NO: 1 of the Sequence Listing, the polypeptide binding to an antibody which is specific to rheumatoid arthritis patients;
(i) a polypeptide including the amino acid sequence shown in SEQ ID NO: 1 of the Sequence Listing, the polypeptide binding to an antibody which is specific to rheumatoid arthritis patients;
(j) a polypeptide including a substitution, deletion or addition in at least one amino acid in the amino acid sequence shown in SEQ ID NO: 1 of the Sequence Listing, the polypeptide binding to an antibody which is specific to rheumatoid arthritis patients;
(k) a polypeptide which is a fragment of the amino acid sequence shown in SEQ ID NO: 3 of the Sequence Listing, the polypeptide binding to an antibody which is specific to rheumatoid arthritis patients;
(l) a polypeptide including the amino acid sequence shown in SEQ ID NO: 3 of the Sequence Listing, the polypeptide binding to an antibody which is specific to rheumatoid arthritis patients; and
(m) a polypeptide including a substitution, deletion or addition in at least one amino acid in the amino acid sequence shown in SEQ ID NO: 3 of the Sequence Listing, the polypeptide binding to an antibody which is specific to rheumatoid arthritis patients.

12. A transformed cell including an expression vector according to claim 10 or 11.

13. A composition for detecting an antibody which is specific to rheumatoid arthritis patients, the composition including a polypeptide binding to an antibody which is specific to rheumatoid arthritis patients.

14. A composition according to claim 13, wherein the composition contains at least one polypeptide selected from a group consisting of the following polypeptides:
(a) a polypeptide shown in SEQ ID NO: 5 of Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to rheumatoid arthritis patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to rheumatoid arthritis patients;
(g) a polypeptide having the amino acid sequence shown in SEQ ID NO: 1 of the Sequence Listing, the polypeptide binding to an antibody which is specific to rheumatoid arthritis patients;
(h) a polypeptide which is a fragment of the amino acid sequence shown in SEQ ID NO: 1 of the Sequence Listing, the polypeptide binding to an antibody which is specific to rheumatoid arthritis patients;
(i) a polypeptide including the amino acid sequence shown in SEQ ID NO: 1 of the Sequence Listing, the polypeptide binding to an antibody which is specific to rheumatoid arthritis patients;
(j) a polypeptide including a substitution, deletion or addition in at least one amino acid in the amino acid sequence shown in SEQ ID NO: 1 of the Sequence Listing, the polypeptide binding to an antibody which is specific to rheumatoid arthritis patients;
(k) a polypeptide which is a fragment of the amino acid sequence shown in SEQ ID NO: 3 of the Sequence Listing, the polypeptide binding to an antibody which is specific to rheumatoid arthritis patients;
(l) a polypeptide including the amino acid sequence shown in SEQ ID NO: 3 of the Sequence Listing, the polypeptide binding to an antibody which is specific to rheumatoid arthritis patients;
(m) a polypeptide including a substitution, deletion or addition in at least one amino acid in the amino acid sequence shown in SEQ ID NO: 3 of the Sequence Listing, the polypeptide binding to an antibody which is specific to rheumatoid arthritis patients; and
(n) a polypeptide having the amino acid sequence shown in SEQ ID NO: 3 of the Sequence Listing, the polypeptide binding to an antibody which is specific to rheumatoid arthritis patients.

15. A composition according to claim 13, wherein the polypeptide is a polypeptide obtained by culturing:
(1) a transformed cell according to claim 12;
(2) a transformed cell including an expression vector including a DNA which encodes a polypeptide having the amino acid sequence shown in SEQ ID NO: 3 of the Sequence Listing and binding to an antibody which is specific to rheumatoid arthritis patients; or
(3) a transformed cell including an expression vector including a DNA shown in SEQ ID NO: 4 of the Sequence Listing.

16. A method for detecting an antibody which is specific to rheumatoid arthritis patients, the method including the steps of: reacting a test sample with a polypeptide binding specifically to an antibody which is specific to the rheumatoid arthritis patient, and detecting a reaction product.

17. A method according to claim 16, wherein she polypeptide is at least one polypeptide selected from a group consisting of the following polypeptides:
(a) a polypeptide shown in SEQ ID NO: 5 of Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to rheumatoid arthritis patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to rheumatoid arthritis patients;
(g) a polypeptide having the amino acid sequence shown in SEQ ID NO: 1 of the Sequence Listing, the polypeptide binding to an antibody which is specific to rheumatoid arthritis patients;
(h) a polypeptide which is a fragment of the amino acid sequence shown in SEQ ID NO: 1 of the Sequence Listing, the polypeptide binding to an antibody which is specific to rheumatoid arthritis patients;
(i) a polypeptide including the amino acid sequence shown in SEQ ID NO: 1 of the Sequence Listing, the polypeptide binding to an antibody which is specific to rheumatoid arthritis patients;
(j) a polypeptide including a substitution, deletion or addition in at least one amino acid in the amino acid sequence shown in SEQ ID NO: 1 of the Sequence Listing, the polypeptide binding to an antibody which is specific to rheumatoid arthritis patients;
(k) a polypeptide which is a fragment of the amino acid sequence shown in SEQ ID NO: 3 of the Sequence Listing, the polypeptide binding to an antibody which is specific to rheumatoid arthritis patients;
(l) a polypeptide including the amino acid sequence shown in SEQ ID NO: 3 of the Sequence Listing, the polypeptide binding to an antibody which is specific to rheumatoid arthritis patients;
(m) a polypeptide including a substitution, deletion or addition in at least one amino acid in the amino acid sequence shown in SEQ ID NO: 3 of the Sequence Listing, the polypeptide binding to an antibody which is specific to rheumatoid arthritis patients; and
(n) a polypeptide having the amino acid sequence shown in SEQ ID NO: 3 of the Sequence Listing, the polypeptide binding to an antibody which is specific to rheumatoid arthritis patients.

18. A kit for detecting an antibody which is specific to rheumatoid arthritis patients, the kit including a polypeptide binding to an antibody which is specific to rheumatoid arthritis patients.

19. A kit according to claim 18, wherein the polypeptide is at least one polypeptide selected from a group consisting of the following polypeptides:
(a) a polypeptide shown in SEQ ID NO: 5 of Sequence Listing;
(b) a polypeptide including the amino acid sequence of SEQ ID NO: 5;
(c) a polypeptide including a substitution, deletion or addition in at least one amino acid in the amino acid sequence shown in SEQ ID NO: 5 of the Sequence Listing, the polypeptide binding to an antibody which is specific to rheumatoid arthritis patients;
(d) a polypeptide shown in SEQ ID NO: 6 of the Sequence Listing;
(e) a polypeptide including the amino acid sequence of SEQ ID NO: 6;
(f) a polypeptide including a substitution, deletion or addition in at least one amino acid in the amino acid sequence shown in SEQ ID NO: 6 of the Sequence Listing, the polypeptide binding to an antibody which is specific to rheumatoid arthritis patients;
(g) a polypeptide having the amino acid sequence shown in SEQ ID NO: 1 of the Sequence Listing, the polypeptide binding to an antibody which is specific to rheumatoid arthritis patients;
(h) a polypeptide which is a fragment of the amino acid sequence shown in SEQ ID NO: 1 of the Sequence Listing, the polypeptide binding to an antibody which is specific to rheumatoid arthritis patients;
(i) a polypeptide including the amino acid sequence shown in SEQ ID NO: 1 of the Sequence Listing, the polypeptide binding to an antibody which is specific to rheumatoid arthritis patients;
(j) a polypeptide including a substitution, deletion or addition in at least one amino acid in the amino acid sequence shown in SEQ ID NO: 1 of the Sequence Listing, the polypeptide binding to an antibody which is specific to rheumatoid arthritis patients;
(k) a polypeptide which is a fragment of the amino acid sequence shown in SEQ ID NO: 3 of the Sequence Listing, the polypeptide binding to an antibody which is specific to rheumatoid arthritis patients;
(l) a polypeptide including the amino acid sequence shown in SEQ ID NO: 3 of the Sequence Listing, the polypeptide binding to an antibody which is specific to rheumatoid arthritis patients;
(m) a polypeptide including a substitution, deletion or addition in at least one amino acid in the amino acid sequence shown in SEQ ID NO: 3 of the Sequence Listing, the polypeptide binding to an antibody which is specific to rheumatoid arthritis patients; and
(n) a polypeptide having the amino acid sequence shown in SEQ ID NO: 3 of the Sequence Listing, the polypeptide binding to an antibody which is specific to rheumatoid arthritis patients.

20. A kit according to claim 18, wherein the polypeptide is a polypeptide obtained by culturing:
(1) a transformed cell according to claim 12;
(2) a transformed cell including an expression vector including a DNA which encodes a polypeptide having the amino acid sequence shown in SEQ ID NO: 3 of the Sequence Listing and binding to an antibody which is specific to rheumatoid arthritis patients; or
(3) a transformed cell including an expression vector including a DNA shown in SEQ ID NO: 4 of the Sequence Listing.
